# EUROPEAN PATENT APPLICATION

(11) **EP 1 702 508 A1**
(43) Date of publication of application: **20.09.2006**
(21) Application number: 05703394.6
(22) Date of filing: 07.01.2005
(51) Int. Cl.: A01H 1/00, A01H 5/00, C12N 15/62

(54) **METHOD OF PRODUCING STERILE PLANT, PLANT OBTAINED BY USING THE SAME AND USE THEREOF**

(30) Priority: 07.01.2004 JP 2004002192; 26.03.2004 JP 2004093796; 29.07.2004 JP 2004221592; 06.08.2004 JP 2004231544
(71) Applicant: Japan Science and Technology Agency, Kawaguchi-shi, Saitama 332-0012 (JP); National Institute of Advanced Industrial Science and Technology, Tokyo 100-8921 (JP)
(72) Inventor: TAKAGI, Masaru, c/o National Institute of Advanced, Tsukuba-shi, Ibaraki 305-8562 (JP); HIRATSU, Keiichiro, National Institute of Advanced, Tsukuba-shi, Ibaraki 305-8562 (JP); MITSUDA, Nobutaka, National Institute of Advanced, Tsukuba-shi, Ibaraki 305-8562 (JP)
(74) Representative: Müller - Hoffmann & Partner
(86) International application number: PCT/JP2005/000155
(87) International publication number: WO 2005/065446

(57) **Abstract**

Transcription of a gene associated with formation of floral organs is suppressed to produce a sterile plant. A plant cell is transfected with a chimeric gene that includes (i) a coding gene of a transcription factor that promotes expression of a gene associated with formation of floral organs, and (ii) a polynucleotide that encodes a functional peptide that converts an arbitrary transcription factor into a transcription repressor, and a chimeric protein in which the transcription factor is fused with the functional peptide is expressed in the plant cell. The expression of the gene associated with formation of floral organs is dominantly suppressed by the chimeric protein, and as a result a male sterile plant is produced that cannot properly form pollen. The chimeric protein also suppresses expression of a gene associated with dehiscence of anther, and as a result a plant is produced in which dehiscence of anther is suppressed. Further, the chimeric protein suppresses expression of target genes of a transcription factor associated with formation of stamen and pistil, and as a result a double flowered plant is produced.

## Description

### TECHNICAL FIELD

The present invention relates to techniques for producing sterile plants, and more specifically to a producing process of male sterile plants, a producing process of plants in which dehiscence of anther is suppressed, a producing process of double flowered plants, plants produced by such processes, and use of the plants.

### BACKGROUND ART

Interbreeding of different varieties produces hybrids that have superior traits to the parents. This is known as heterosis. Today, heterosis is commonly employed in interbreeding to produce superior varieties in the hybrid crops. For example, in major vegetables and cereals, most of the superior varieties have been improved by such interbreeding.

Heterosis requires interbreeding of different varieties. As such, there is a need to prevent self-pollination in crossing different varieties. In plants in which male and female flowers occur separately as in corn, self-pollination can be avoided by cutting the male flowers. However, this is labor intensive and time consuming. In self-fertilizing plants as represented by rice, a large number of flowers aggregate together, and the stamen and pistil often surround the petals. This poses difficulty in removing the male organ (or stamen), making it extremely difficult to avoid self-pollination.

It is therefore desirable to use a so-called male sterile plant which cannot form pollens properly for heterotic interbreeding. In fact, male sterile plants have been produced and used for improvement in many plants, including tomatoes and cucumbers.

In many other plants, such male sterile plants are yet to be produced. If male sterility were to be newly produced by mutation in these plants, then the success depends solely on chances, and the interbreeding takes a long time. For the large cost and labor it requires, this approach is not practical in contemporary agriculture.

Under these circumstances, there have been attempts to artificially establish male sterility using genetic recombinant techniques.

Non-Patent Document 1 discloses a technique in which male sterility is brought about by manipulating nuclear genes. In this technique, the anti-sense gene of Chalcon-synthase is introduced into *Petunia* to suppress activities of Chalcon-synthase. This suppresses the biosynthesis of Chalcon, which is a biosynthetic precursor of flavonoid, and thereby transform the plant into a male sterile plant.

Non-Patent Document 2 discloses a technique in which male sterility is introduced by eliminating the tapetal tissue of tobacco with a toxic substance. In this technique, argE gene that is transcribed and translated into N-acetyl-L-ornithine decarboxylase is introduced into a tobacco by fusing it with a DNA sequence that resembles the TA29 promoter whose activities are specific to the tapetal tissue. The tobacco is then administered with non-toxic N-acetyl-L-phosphinoslysine. The N-acetyl-L-phosphinoslysine is deacetylated by the N-acetyl-L-ornithine decarboxylase expressed in the anther, and toxic L-phosphinoslysine is produced. The toxic L-phosphinoslysine causes necrosis and eliminates the tapetal tissue, with the result that the transformed tobacco becomes a male sterile plant that produces no pollen.

Non-Patent Document 3 discloses a technique of causing cytoplasmic male sterility. In this technique, wheat mitochondrion atp9 gene with no RNA editing is introduced into a tobacco. As a result, inactive ATP9 protein is expressed and the protein moves into the mitochondria. This inhibits the functions of the mitochondria and thereby introduces male sterility in the transformed tobacco.

Non-Patent Document 4 discloses a technique in which a transformed tobacco is produced that has incorporated the anti-sense gene of mitochondrion atp9 gene that has no RNA editing, and the transformed tobacco is crossed with another transformed tobacco that has incorporated mitochondrion atp9 gene that has no RNA editing. In this way, the progeny of the cross recovers fertility.

Conventionally, the NAC family, one of the plant specific transcription factor families has been known in plants. In *Arabidopsis thaliana,* more than 100 genes have been reported that belong to the NAC family. Further, there have been reports that some of the isolated members of the NAC family are transcription factors that are necessary for the formation and sustainment of shoot apical meristem, and the formation of floral organs and lateral roots, for example. Various other functions of the NAC family have been revealed. However, no information is available as to the cis sequence or the like which the NAC family specifically binds to, and further functional analyses is sought for (see Non-Patent Document 5, for example).

Today, interbreeding of different plant varieties has been commonly carried out to produce hybrid crops of superior properties. This takes advantage of heterosis, which describes the tendency of the progeny of a cross between different plant varieties to outperform the parents. Heterosis requires interbreeding of different varieties. Therefore, there is a need to prevent self-pollination in crossing different varieties. This can be achieved, for example, by removing male organs by hand, artificially crossing the plants, or inhibiting maturation of pollens with chemicals. However, these methods are labor intensive and time consuming, and are not applicable to all plants. An alternative method that is pervasive nowadays is a method using male sterile plants which cannot form complete male gametes (pollens) and therefore cannot form viable seeds. Today, male sterile plants obtained by mutation are used for the improvement of a wide variety of plants. However, since male sterile plants have not been established in many of the plant species, attempts have been made to artificially establish male sterility using genetic recombinant techniques (see Patent Documents 1 and 2, for example). Patent Document 1 discloses a method by which male sterile plants are produced by controlling expression of DAD1 gene that controls dehiscence of anther and maturation of pollen.

Meanwhile, there has been an attempt to suppress dehiscence of anther whereby cytotoxic barnase is ligated to TA56 promoter that causes anther-specific gene expression, and the construct is introduced into a tobacco to kill off the stomium cells in the anther (see Non-Patent Document 6, for example). As reported, this is said to inhibit dehiscence of anther. Further, Non-Patent Document 6 reports that, in order for the dehiscence to occur, the stomium cells need to remain sufficiently functional until they are killed. However, little is known about the dehiscence of anther at molecular level. A relatively large number of mutants with abnormal anther dehiscence are known, including delayed-dehiscence 1 through delayed-dehiscence 5, non-dehiscence 1, and msH, for example (see Non-Patent Document 7, for example). However, only a handful of genes, including DAD1, are known as the causal genes that control anther dehiscence, and many other causal genes are yet to be found.

Further, there is a report that corn transposon En-1/Spm was used to induce mutation in a population of *Arabidopsis thaliana* and mutants with abnormal anther dehiscence were isolated for the identification of causal genes (see Non-Patent Document 8, for example). As reported in Non-Patent Document 8, the phenotype of the mutants is conferred by the insertion of the transposon in AtMYB26.

Generally, a flower is made up of 4 organs: the sepal, petal, stamen, and pistil. The meristem of a flower bud can be divided into 4 concentric regions, whorl 1 to whorl 4, from outside toward the center. In this case, whorl 1 includes four sepals, whorl 2 includes four petals, whorl 3 includes six stamens, and whorl 4 includes one pistil that is made up of two fused carpels. The traits of these organs are known to be determined by the homeotic genes of plants (see Non-Patent Document 9, for example). The homeotic genes of plants are categorized into 3 classes, A, B, and C, and encode transcription factors. The type of floral organ formed in the meristem of the flower bud is specified by combinations of these classes of genes. Specifically, the specification of sepals is dependent on class A gene activities. Petals are specified by a combination of class A and class B gene activities, stamens are specified by the combined activities of classes B and C, and specification of the pistil is achieved by class C activity. Thus, if activities of the homeotic genes are lost by mutation for example, transformation of one organ into another occurs. Such a change is known as homeotic conversion, and, in plants, homeotic genes that are associated with specification of floral organs have been identified in *Arabidopsis thaliana* and other plants (see Non-Patent Document 9, for example).

One member of class C genes is AGAMOUS gene (hereinafter referred to as "AG gene"). AG mutants lacking AG gene activities are known to produce double flowers, with the class A activities affecting the entire region of the flower to change the stamen into the petal and form a new flower in regions where pistils are normally formed in the wild type (see Non-Patent Document 9, for example).

In order to change morphology of flowers for the purpose of producing double flowered plants, it is common to cross different varieties of desired traits.

Further, as a method of suppressing activities of a specific gene, the RNA interference (RNAi) method has become common in which double strand RNA is introduced into a cell to degrade mRNA that is complementary to the sequence of the double strand RNA.

Meanwhile, the inventors of the present invention have found a variety of peptides that convert arbitrary transcription factors into transcription repressors (see Patent Documents 3 to 9, Non-Patent Documents 10 and 11, for example). These peptides were excised from Class II ERF (Ethylene Responsive Element Binding Factor) proteins, or plant zinc finger proteins (for example, *Arabidopsis thaliana* SUPERMAN protein), and therefore have very simple structures.

Further, the inventors of the present invention have attempted to transfect a plant with a gene that encodes a fusion protein (chimeric protein) in which the transcription factor is fused with the peptide. As a result, the transcription factor was successfully converted into a transcription repressor, and a plant was produced in which expression of target genes of the transcription factor is suppressed.

### [Patent Document 1]

Japanese Laid-Open Patent Publication No. 300273/2000 (Tokukai 2000-300273, published on October 31, 2000)

### [Patent Document 2]

Japanese Laid-Open Patent Publication No. 24108/2004 (Tokukai 2004-24108, published on January 29, 2004)

### [Patent Document 3]

Japanese Laid-Open Patent Publication No. 269177/2001 (Tokukai 2001-269177, published on October 2, 2001)

### [Patent Document 4]

Japanese Laid-Open Patent Publication No. 269178/2001 (Tokukai 2001-269178, published on October 2, 2001)

### [Patent Document 5]

Japanese Laid-Open Patent Publication No. 292776/2001 (Tokukai 2001-292776, published on October 2, 2001)

### [Patent Document 6]

Japanese Laid-Open Patent Publication No. 292777/2001 (Tokukai 2001-292777, published on October 23, 2001)

### [Patent Document 7]

Japanese Laid-Open Patent Publication No. 269176/2001 (Tokukai 2001-269176, published on October 2, 2001)

### [Patent Document 8]

Japanese Laid-Open Patent Publication No. 269179/2001 (Tokukai 2001-269179, published on October 2, 2001)

### [Patent Document 9]

International Publication No. WO03/055903, pamphlet (published on July 10, 2003)

### [Non-Patent Document 1]

Ingrid M. van der Meer, Maike E. Stam, Arjen J. van Tunen, Joseph N. M. Mol, and Antoine R. Stuitje. The Plant Cell, Vol 4, pp 253-262, March, 1992

### [Non-Patent Document 2]

G. Kriete, K. Niehaus, A.M. Perlick, A. Puehler and I. Broer, The Plant Journal, Vol 9, pp 809-818, 1996

### [Non-Patent Document 3]

Michel Hernould, Sony Suharsono, Simon Litvak, Alejandro Araya, and Armand Mouras., Proc. Natl. Acad. Sci. USA, Vol 90, pp. 2370-2374, March, 1993

### [Non-Patent Document 4]

Eduardo Zabaleta, Armand Mouras, Michel Hernould, Suharsono, and Alejandro Araya., Proc. Natl. Acad. Scl. USA, Vol 93, pp 11259-11263, October, 1996

### [Non-Patent Document 5]

Xie,Q.,Frugis,G.,Colgan,D.,Chua,N-H., Genes Dev.14,3024-3036,2000

### [Non-Patent Document 6]

Beals,T.P.,Goldberg,R.B., The Plant Cell, Vol.9,1527-1545,September, 1997

### [Non-Patent Document 7]

Flowers-Molecular Biology of Sex and Reproduction, Yasuyoshi Hyuga, Japan Scientific Society Press, pp. 116-117

### [Non-Patent Document 8]

Steiner-Lange, S., Unte,U.S.,Eckstein,L.,Yang,C.,Wilson,Z.A .,Schmelzer,E.,Dekker,K.,Saedler,H., The Plant Journal(2003)34,519-528

### [Non-Patent Document 9]

Molecular Mechanisms as a Determinant of Plant Shape, Hajime Sakai, Shujunsha, pp. 150-155

### [Non-Patent Document 10]

Ohta, M., Matsui, K., Hiratsu, K., Shinshi, H. and Ohme-Takagi, M., The Plant Cell, Vol.13, 1959-1968, August, 2001

### [Non-Patent Document 11]

Hiratsu, K., Ohta,M., Matsui, K., Ohme-Takagi, M., FEBS Letters 514(2002)351-354

However, no technique is available that produces a sterile plant by suppressing transcription of a gene associated with formation of floral organs.

In Patent Document 1, dehiscence of anther is inhibited and pollens are sterile. However, regardless of whether the pollens are sterile, self-pollination can be avoided in the heterotic interbreeding if dehiscence of anther were prevented.

Further, the conventional method using male sterile plants that produce no pollen is effective in producing a hybrid first filial generation because it automatically forms hybrids when the plants form seeds. However, since the seeds will not be formed if the next generation is sterile, fertility of the plants needs to be recovered in the next generation. In this connection, if dehiscence of anther were suppressed and pollens were fertile, then it would be possible to produce a plant that can produce viable pollens yet does not cause self-pollination. This would be highly useful in interbreeding. To this date, no technique is available that suppresses dehiscence of anther by producing a chimeric protein of (i) a transcription factor that promotes transcription of a gene associated with dehiscence of anther, and (ii) a functional peptide that converts the transcription factor into a transcription repressor.

Further, the conventional interbreeding takes years and requires the skill of an experience person to produce a plant of desired traits. It is therefore very difficult to easily and reliably produce a double flowered plant in a short time period.

Double flowered plants can be produced by suppressing AG gene activities by the RNAi method. However, the RNAi method has various problems. One problem is that it requires trial and error due to the difficulty in determining the target sites when suppressing gene expression. Other problems include difficulties in constructing constructs, and the limited effect of RNA interference due to poor transfection efficiency of some cells. It is therefore very difficult to easily and reliably produce double flowered plants by the RNAi method in a short time period.

The present invention was made in view of the foregoing problems, and an object of the present invention is to provide a producing process of a sterile plant, whereby a sterile plant is produced by suppressing a transcription factor of a gene associated with formation of floral organs.

Another object of the present invention is to provide a producing process of a sterile plant in which dehiscence of anther is suppressed, whereby a chimeric protein of (i) a transcription factor that promotes transcription of a gene associated with dehiscence of anther, and (ii) a functional peptide that converts the transcription factor into a transcription repressor is produced.

Yet another object of the present invention is to provide a producing process of a sterile plant, whereby double flowered plants are easily and reliably produced in a short time period by converting a transcription factor associated with formation and stamen and pistil into a transcription repressor and thereby suppressing transcription of target genes of the transcription factor.

### DISCLOSURE OF INVENTION

The inventors of the present invention diligently worked to solve the foregoing problems, and accomplished the invention by finding that a male sterile plant with no petal and stamen can be produced by converting APETALA3 protein, one of the transcription factors that promotes transcription of a gene associated with formation of floral organs, into a transcription repressor.

Specifically, a producing process of a sterile plant according to the present invention includes causing a plant to produce a chimeric protein, in which a transcription factor that promotes expression of a gene associated with formation of floral organs is fused with a functional peptide that converts an arbitrary transcription factor into a transcription repressor, so as to sterilize the plant.

Further, a producing process of a sterile plant according to the present invention includes causing a plant to produce a chimeric protein, in which a transcription factor that promotes expression of a gene associated with formation of floral organs is fused with a functional peptide that converts an arbitrary transcription factor into a transcription repressor, so as to suppress expression of the gene associated with formation of floral organs.

It is preferable in the producing process of a sterile plant according to the present invention that the transcription factor that promotes expression of a gene associated with formation of floral organs be a transcription factor associated with formation of stamen or pistil.

According to the foregoing arrangement, the resulting plant is sterile and does not produce seeds. Thus, a sterile plant can be obtained very easily without using complicated genetic recombinant techniques.

It is preferable in the producing process of a sterile plant according to the present invention that at least formation of stamen be suppressed in the sterile plant.

The inventors of the present invention diligently worked to solve the foregoing problems, and found that dehiscence of anther in a plant does not occur either completely or occurs only incompletely if a protein that is encoded by At2g46770 gene locus, a member of the NAC family protein, were fused with a peptide that converts an arbitrary transcription factor into a transcription repressor, and the fusion protein so prepared were expressed in the plant. Based on this finding, the inventors revealed, for the first time, that the NAC family protein (protein encoded by At2g46770 gene locus, hereinafter may be referred to as "NACAD1" (NAC involving to Anther Development)) is a transcription factor that promotes transcription of a gene associated with dehiscence of anther, and thereby accomplished the present invention. That is, it is preferable in the producing process of a sterile plant according to the present invention that the transcription factor associated with formation of stamen or pistil be a transcription factor that promotes transcription of a gene associated with dehiscence of anther, and that a chimeric protein in which the transcription factor is fused with a functional peptide that converts an arbitrary transcription factor into a transcription repressor be produced in a plant so as to suppress dehiscence of anther.

The inventors of the present invention diligently worked to solve the foregoing problems, and found that dehiscence of anther in a plant does not occur either completely or occurs only incompletely if the MYB26 protein, which is a transcription factor that promotes transcription of a gene associated with dehiscence of anther, and which includes an MYB domain, were fused with a peptide that converts an arbitrary transcription factor into a transcription repressor, and the fusion protein so prepared were expressed in the plant. Based on this finding, the inventors accomplished the present invention.

That is, it is preferable in the producing process of a sterile plant according to the present invention that the transcription factor that promotes transcription of a gene associated with dehiscence of anther be a transcription factor with an MYB domain, and that a chimeric protein in which the transcription factor is fused with a functional peptide that converts an arbitrary transcription factor into a transcription repressor be produced in a plant so as to suppress transcription of the gene associated with dehiscence of anther. It is preferable that the plant have sterile female organs. It is preferable that the plant produce sterile pollens.

In this way, the chimeric protein can effectively suppress transcription of target genes of the transcription factor. As a result, dehiscence of anther can be suppressed in the plant in which the chimeric protein is produced.

The inventors of the present invention revealed, for the first time, that a double flowered plant can be easily and reliably produced in a short time period if the transcription factor that is associated with formation of stamen and pistil were converted into a transcription repressor, and transcription of the target genes of the transcription factor were suppressed with the transcription repressor. Based on this finding, the inventors accomplished the present invention.

That is, a producing process of a sterile plant according to the present invention includes causing a plant to produce a chimeric protein, in which a transcription factor associated with formation of stamen and pistil is fused with a functional peptide that converts an arbitrary transcription factor into a transcription repressor, so as to produce a double-flowered plant.

According to the foregoing arrangement, the transcription factor is converted into a transcription repressor, and transcription of the target genes of the transcription factor is suppressed in the plant. Further, since the trait that suppresses the transcription of the target genes of the transcription factor is dominant, the chimeric protein acts dominantly over the transcription factor to suppress transcription of the target genes. It is therefore possible to conveniently and reliably produce double flowered plants in a short time period.

Further, the double flowered plant produced by the present invention is sterile and does not form seeds. It is therefore possible to easily obtain a sterile plant without using complicated genetic recombinant techniques.

The producing process of a sterile plant according to the present invention may include a transforming step of introducing into plant cells a recombinant expression vector that includes a chimeric gene containing (i) a coding gene of the transcription factor and (ii) a polynucleotide that encodes the functional peptide.

Further, the producing process of a sterile plant according to the present invention may include an expression vector constructing step of constructing the recombinant expression vector.

According to the foregoing arrangement a gene of a transcription factor is introduced into a cassette vector that has incorporated the functional peptide, and the cassette vector is then introduced into plant cells. With such a simple procedure, the chimeric protein can be expressed in the plant cell, and the transcription of the target genes of the transcription factor can easily be suppressed with the chimeric protein. It is therefore possible to conveniently and reliably produce sterile plants in a short time period.

The transcription factor is:
- (a): a protein with an amino acid sequence represented by SEQ ID NO: 134, or
- (b): a protein with the substitution, deletion, insertion, and/or addition of one to several amino acids in the amino acid sequence represented by SEQ ID NO: 134, and capable of promoting expression of the gene associated with formation of floral organs.

The coding gene of the transcription factor is preferably:
- (c): a gene that has a base sequence of SEQ ID NO: 135 as an open reading frame; or
- (d): a gene that hybridizes under stringent conditions with a gene of a base sequence complementary to the gene of the base sequence represented by SEQ ID NO: 135, and that encodes the transcription factor that promotes expression of the gene associated with formation of floral organs.

The transcription factor is preferably:
- (a): a protein with an amino acid sequence represented by SEQ ID NO: 136; or
- (b): a protein with the substitution, deletion, insertion, and/or addition of one to several amino acids in the amino acid sequence represented by SEQ ID NO: 136, and capable of promoting transcription of a gene associated with dehiscence of anther.

The transcription factor may share 50% or greater homology with the amino acid sequence of SEQ ID NO: 136, and may be a protein capable of promoting transcription of a gene associated with dehiscence of anther.

The coding gene of the transcription factor is preferably:
- (c): a gene that has a base sequence of SEQ ID NO: 137 as an open reading frame; or
- (d): a gene that hybridizes under stringent conditions with a gene of a base sequence complementary to the gene of the base sequence represented by SEQ ID NO: 137, and that encodes a transcription factor that promotes transcription of a gene associated with dehiscence of anther.

The transcription factor is preferably:
- (a): a protein with an amino acid sequence represented by SEQ ID NO: 138; or
- (b): a protein with the substitution, deletion, insertion, and/or addition of one to several amino acids in the amino acid sequence represented by SEQ ID NO: 138, and capable to promoting transcription of a gene associated with dehiscence of anther.

The coding gene of the transcription factor is preferably:
- (c): a gene that has a base sequence of SEQ ID NO: 139 as an open reading frame; or
- (d): a gene that hybridizes under stringent conditions with a gene of a base sequence complementary to the gene of the base sequence represented by SEQ ID NO: 139, and that encodes a transcription factor that promotes transcription of a gene associated with dehiscence of anther.

The transcription factor is preferably:
- (a): a protein with an amino acid sequence represented by SEQ ID NO: 140; or
- (b): a protein with the substitution, deletion, insertion, and/or addition of one to several amino acids in the amino acid sequence represented by SEQ ID NO: 140.

The coding gene of the transcription factor is preferably:
- (c): a gene that has a base sequence of SEQ ID NO: 141 as an open reading frame; or
- (d): a gene that hybridizes under stringent conditions with a gene of a base sequence complementary to the gene of the base sequence represented by SEQ ID NO: 141, and that encodes a protein associated with formation of stamen and pistil.

According to the foregoing arrangement, the transcription factor is converted into a transcription repressor with the functional peptide, and transcription of the target genes of the transcription factor is suppressed. As a result, a double flowered plant can be easily and reliably produced in a short time period.

The present invention provides a producing process of a plant in which dehiscence of anther is suppressed, and the process uses a gene that encodes:
- (a): a protein with an amino acid sequence represented by SEQ ID NO: 136; or
- (b): a protein with the substitution, deletion, insertion, and/or addition of one to several amino acids in the amino acid sequence represented by SEQ ID NO: 136, and capable to promoting transcription of a gene associated with dehiscence of anther,
or alternatively, the process uses:
- (c): a gene that has a base sequence of SEQ ID NO: 137 as an open reading frame; or
- (d): a gene that hybridizes under stringent conditions with a gene of a base sequence complementary to the gene of the base sequence represented by SEQ ID NO: 137.

The functional peptide preferably has an amino acid sequence represented by one of:
- (1): X1-Leu-Asp-Leu-X2-Leu-X3, where X1 represents 0 to 10 amino acid residues, X2 represents Asn or Glu, and X3 represents at least 6 amino acid residues;
- (2): Y1-Phe-Asp-Leu-Asn-Y2-Y3, where Y1 represents 0 to 10 amino acid residues, Y2 represents Phe or Ile, and Y3 represents at least 6 amino acid residues;
- (3): Z1-Asp-Leu-Z2-Leu-Arg-Leu-Z3, where Z1 represents Leu, Asp-Leu, or Leu-Asp-Leu, Z2 represents Glu, Gln, or Asp, and Z3 represents 0 to 10 amino acid residues; and
- (4): Asp-Leu-Z4-Leu-Arg-Leu, where Z4 is Glu, Gln, or Asp.

The functional peptide preferably has an amino acid sequence represented by any one of SEQ ID NO: 1 though 17.

The functional peptide may be:
- (e): a peptide with an amino acid sequence represented by SEQ ID NO: 18 or 19; or
- (f): a peptide with the substitution, deletion, insertion, and/or addition of one to several amino acids in the amino acid sequence represented by SEQ ID NO: 18 or 19.

Further, the functional peptide may have an amino acid sequence represented by:

α1-Leu-β1-Leu-γ1-Leu (5)

where α1 is Asp, Asn, Glu, Gln, Thr, or Ser, β1 is Asp, Gln, Asn, Arg, Glu, Thr, Ser, or His, and γ1 is Arg, Gln, Asn, Thr, Ser, His, Lys, or Asp.

The functional peptide may have an amino acid sequence represented by:

α1-Leu-β1-Leu-γ2-Leu (6)

α1-Leu-β2-Leu-Arg-Leu (7)

α2-Leu-β1-Leu-Arg-Leu (8)

where α1 is Asp, Asn, Glu, Gln, Thr, or Ser, α2 is Asn, Glu, Gln, Thr, or Ser, β1 is Asp, Gln, Asn, Arg, Glu, Thr, Ser, or His, β2 is Asn, Arg, Thr, Ser, or His, and γ2 is Gln, Asn, Thr, Ser, His, Lys, or Asp.

Further, the functional peptide may be a peptide with an amino acid sequence represented by SEQ ID NO: 20, 21, 22, 23, 24, 25, 26, 27, 28, 29, 30, 31, 32, 33, 34, 35, 38, 39, 40, or 152.

Further, the functional peptide may be a peptide with an amino acid sequence represented by SEQ ID NO: 36 or 37.

The functional peptide is a peptide with an amino acid sequence as represented by any one of the foregoing formulae, or any one of the SEQ ID NOs: set forth above. Since most of these peptides are extremely short and are therefore easy to synthesize. Thus, transcription of the target genes of the transcription factor can be suppressed efficiently. Further, since the functional peptide preferentially suppresses the expression of the target genes over the activities of other functionally redundant transcription factors, the expression of the target genes can be effectively suppressed.

A plant according to the present invention is a sterile plant which is produced by the producing processes. It is preferable that the sterile plant include at least one of: an adult plant; a plant cell; a plant tissue; a callus; and a seed.

A sterile plant producing kit according to the present invention is for performing the foregoing producing process, and the kit includes a recombinant expression vector that includes: a gene that encodes a transcription factor that promotes expression of a gene associated with formation of floral organs, formation of stamen or pistil, dehiscence of anther, or formation of stamen and pistil; a polynucleotide that encodes a functional peptide that converts an arbitrary transcription factor into a transcription repressor; and a promoter. The sterile plant producing kit may further include chemicals for introducing the recombinant expression vector into plant cells.

For a fuller understanding of the nature and advantages of the invention, reference should be made to the ensuing detailed description taken in conjunction with the accompanying drawings.

### BRIEF DESCRIPTION OF DRAWINGS

Figure 1(a) is a whole view of adult *Arabidopsis thaliana* transformed with p35S::APETALA3SRDX in Example.
Figure 1(b) is an enlarged view showing a tip of the adult *Arabidopsis thaliana* transformed with p35S::APETALA3SRDX in Example.
Figure 1(c) is an enlarged view showing floral organs of *Arabidopsis thaliana* transformed with p35S::APETALA3SRDX in Example.
Figure 2 is a diagram illustrating the steps of constructing a construction vector for constructing a recombinant expression vector used in Example.
Figure 3 is a diagram illustrating the steps of inserting a coding gene of a transcription repressor converting peptide SRDX and NACAD1 gene into construction vector p35SG used in Example.
Figure 4 is a diagram illustrating the steps of inserting a coding gene of transcription repressor converting peptide SRDX and MYB26 gene into construction vector p35SG used in Example.
Figure 5 is a diagram illustrating the steps of inserting a coding gene of transcription repressor converting peptide SRDX and AG gene into construction vector p35SG used in Examples.
Figure 6 is a diagram illustrating the steps of constructing transformation vector pBIGCKH.
Figure 7(a) is a view showing a shape of anther of *Arabidopsis thaliana* transformed with recombinant expression vector pBIG-NACAD1SRDX in Example.
Figure 7(b) is a view showing a shape of anther of wild type *Arabidopsis thaliana.*
Figure 8 is a view showing *Arabidopsis thaliana* transformed with recombinant expression vector pBIG-NACAD1SRDX in Example (right-hand side), and wild type *Arabidopsis thaliana* (left-hand side).
Figure 9(a) is a graph showing the number of individuals of *Arabidopsis thaliana* transformed with recombinant expression vector pBIG-NACAD1SRDX in Example, plotted against the class values given by (the weight of harvested seeds × 100/the dry weight of non-seed ground part).
Figure 9(b) is a graph showing the number of individuals of wild type *Arabidopsis thaliana,* plotted against the class values given by (the weight of harvested seeds × 100/the dry weight of non-seed ground part).
Figure 10 is a view showing a result of evaluation that was performed in Example to see if a plant that was transformed with pBIG-NACAD1SRDX and that has had dehiscence of anther suppressed has the ability to form seeds when pollinated with the pollens removed from the anther.
Figure 11 (a) is a view showing a shape of anther of wild type *Arabidopsis thaliana.*
Figure 11 (b) is a view showing a shape of anther of *Arabidopsis thaliana* transformed with recombinant expression vector pBIG-MYB26SRDX in Example.
Figure 12(a) is a graph showing the number of individuals of wild type *Arabidopsis thaliana,* plotted against the class values given by ((the number of hulls with seeds/the number of flowers) ×100).
Figure 12(b) is a graph showing the number of individuals of *Arabidopsis thaliana* transformed with recombinant expression vector pBIG-MYB26SRDX in Example, plotted against the class values given by ((the number of hulls with seeds/the number of flowers) ×100).
Figure 13(a) is a view showing a flower of completely double flowered *Arabidopsis thaliana* transformed with pBIG-AGSRDX.
Figure 13(b) is a view showing a whole part of double flowered *Arabidopsis thaliana.*
Figure 14(a) is a view showing a flower of wild type *Arabidopsis thaliana.*
Figure 14(b) is a view showing a flower of AG mutant *Arabidopsis thaliana.*
Figure 15 is a view showing a flower of incompletely double flowered *Arabidopsis thaliana* transformed with recombinant expression vector pBIG-AGSRDX.
Figure 16 is a view showing a flower of *Arabidopsis thaliana* that was transformed with recombinant expression vector pBIG-AGSRDX and resembles the wild type.

### BEST MODE FOR CARRYING OUT THE INVENTION

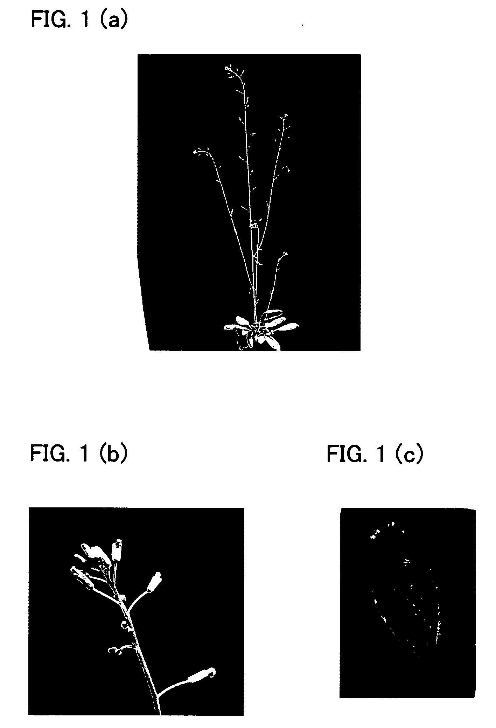

Referring to Figure 1(a) through Figure 16, the following will describe one embodiment of the present invention. It should be noted that the invention is not limited in any way by the following description.

The present invention is a technique for producing a sterile plant by causing a plant to produce a chimeric protein in which a transcription factor that promotes expression of a gene associated with formation of floral organs is fused with a functional peptide that converts an arbitrary transcription factor into a transcription repressor. Since the plant produced in this manner cannot properly form pollens, the present invention can produce a male sterile plant.

Normal pollen formation is inhibited according to the following scheme. First, the transcription factor DNA binding domain of the chimeric protein binds to possible target genes associated with formation of floral organs. In response, the transcription factor is converted into a transcription repressor, and transcription of the target genes is suppressed. This inhibits formation of stamen or other organs, and a male sterile plant is produced that cannot form pollens properly.

A male sterile plant produced by a producing process of the present invention (present male sterile plant) cannot properly form pollens. Specifically, the present male sterile plants include those which cannot form pollens due to the inhibited formation of stamen, those with the stamen but which cannot form pollens due to the absence of anther, and those with the stamen and anther but which cannot cause dehiscence of anther due to the small amount of pollen they produce, and those which cannot spread pollens due to enlargement and fusion of pollens.

In the present male sterile plant, the pistil is fertile. Thus, the present male sterile plant can receive pollens from other plant species. This enables the first filial generation to be produced by hetrotic breeding.

Further, the present male sterile plant that cannot properly form pollens is not necessarily required to properly form other tissues. For example, in the present male sterile plant, the shape of petal or sepal may be abnormal, or these and other organs may not be formed at all. For example, by the absence of petals and sepals, the pistil is exposed. This simplifies the procedure of pollinating the plant with the pollens of other plant species (for example, removal of sepals and petals can be omitted).

The present invention is a technique for suppressing dehiscence of anther, and produces a sterile plant by causing a plant to produce a chimeric protein in which a transcription factor that promotes expression of a gene associated with dehiscence of anther is fused with a functional peptide that converts an arbitrary transcription factor into a transcription repressor. Since the plant produced in this manner can suppress transcription of a gene associated with dehiscence of anther, the present invention can produce a plant in which dehiscence of anther is suppressed.

Dehiscence of anther is suppressed according to the following scheme. First, the transcription factor DNA binding domain of the chimeric protein binds to possible target genes associated with dehiscence of anther. In response, the transcription factor is converted into a transcription repressor, and transcription of the target genes is suppressed. This reduces the amount of possible protein associated with dehiscence of anther, and dehiscence of anther can be suppressed in the plant.

The present invention is a technique for suppressing dehiscence of anther and causes a plant to produce a chimeric protein in which a transcription factor that promotes expression of a gene associated with dehiscence of anther and that has MYB domain is fused with a functional peptide that converts an arbitrary transcription factor into a transcription repressor. Since the plant produced in this manner can suppress transcription of a gene associated with dehiscence of anther, the present invention can produce a plant in which dehiscence of anther is suppressed.

A common RNAi method can be used to suppress gene activities, for example. However, due to the poor transfection efficiency in some cells, the effect of the method is often limited. Thus, the RNAi method poses difficulties in determining target sites and requires trial and error. Construction of constructs is also difficult in the RNAi method. In the method according to the present invention, a plant is transfected with a chimeric gene in which a coding gene of the transcription factor is ligated to a coding gene of the functional peptide. In this way, dehiscence of anther can very easily be suppressed in a target plant.

A producing process according to the present invention produces a plant in which dehiscence of anther is suppressed. In the plant, dehiscence of anther does not occur completely, or occurs only incompletely. As used herein, the "anther" is a part of stamen and it comprises a sac where pollens are formed. In plants that undergo cleistogamy (for example, *Leguminosae*), the pollen germinates in the anther, and the pollen tube extends through a soft anther wall where fibrous cell layer is not formed. However, more commonly, the anther dehisces to release the pollen. Dehiscence of anther occurs as the anther wall cleaves in portions where the stomium cells are killed. The cleaving of the stomium cells is not enough to open the anther, and the pollens are not released until the anther wall contracts to stretch itself. As used herein, the "plants in which dehiscence of anther is suppressed" include plants in which the stomium cells are cleaved, and plants in which the stomium cells cleaves and the anther wall is stretched to open the anther. Note that, the stomium cells make up single-layer cell tissues where the anther opens.

In a plant in which dehiscence of anther is suppressed according to the present invention, the female organ (pistil) is fertile. Thus, the plant can be pollinated with the pollens of other plant species. This enables the first filial (F1) generation to be produced by hetrotic breeding, without the need to remove the male organ or artificially crossing the plants.

Further, in a plant in which dehiscence of anther is suppressed according to the present invention, the pollen itself may be fertile or not fertile, as long as dehiscence of anther is suppressed. However, it is preferable that the pollen be fertile. In this way, the plant forms viable pollens yet does not cause self-pollination. This is useful in interbreeding. Specifically, a male sterile plant that produces infertile pollens is incapable of producing and sustaining homozygous progeny by self-fertilization because the pollens are infertile. Homozygous plants can be produced by self-pollinating heterozygous individuals; however, in this case, only 1/4 of the progeny will be homozygous. On the contrary, homozygous individuals can be produced and sustained if pollens are fertile. The present invention is therefore applicable to breeding.

The present invention is a technique for producing a double flowered plant by causing a plant to produce a chimeric protein in which a transcription factor that promotes expression of a gene associated with formation of stamen and pistil is fused with a functional peptide that converts an arbitrary transcription factor into a transcription repressor.

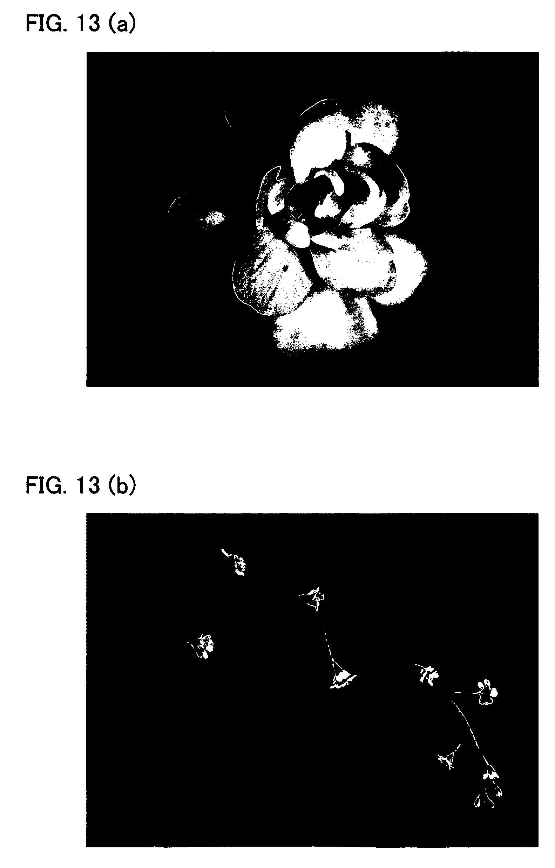

In the method producing the chimeric protein, the transcription factor is converted into a transcription repressor with the functional peptide. Thus, when the transcription factor DNA binding domain of the chimeric protein binds to target genes of the transcription factor, transcription of the target genes is suppressed. As a result, activities of the gene associated with formation of stamen and pistil are lost, and the class A activities affect the entire region of the flower to change the stamen into the petal and form a new flower in regions where pistils are normally formed in the wild type. The result is a double flowered plant. As shown in Figure 13(a), the flower includes the sepal, petal, and petal in this order toward the center.

The conferred trait of the chimeric protein to suppress transcription of target genes of the transcription factor is dominant. That is, the mutant gene that suppresses transcription of target genes of the transcription factor is expressed dominantly over the normal gene associated with formation of stamen and pistil. In other word, the chimeric protein acts dominantly over the transcription factor to suppress transcription of target genes.

In this way, a double flowered plant can be easily and reliably produced in a short time period. The double flowered plant is sterile and does not form seeds. The "sterile plants" include not only completely sterile plants lacking stamen and pistil but also sterile plants which form incomplete stamen-like organ and/or pistil-like organ yet cannot form seeds.

The sterile plants do not form seeds, and the pollen does not spread in the completely sterile plants. Thus, the genetically recombinant plants can be prevented from spreading in the environment.

In the following, description is made as to a chimeric protein used in a producing process of a sterile plant according to the present invention, an exemplary plant producing process, a plant produced by such a process, and usefulness and use of the plant.

### (I) Chimeric Protein used in the Invention

As described above, in a chimeric protein used in the present invention, a transcription factor that promotes transcription of a gene associated with formation of floral organs, formation of stamen or pistil, dihescence of anther, or formation and stamen and pistil is fused with a functional peptide which converts an arbitrary transcription factor into a transcription repressor. The transcription factor that promotes transcription of a gene associated with dihescence of anther may be a transcription factor with the MYB domain.

Further, a chimeric protein used in the present invention acts dominantly over endogenous genes. Specifically, a chimeric protein according to the present invention exhibits the same repressing action on the expression of a gene associated with formation of floral organs, formation of stamen or pistil, dehiscence of anther, or formation and stamen, regardless of whether the plant is a diploid or an amphiploid, or the plant have functionally redundant genes. Thus, any transfectable plant can easily be transformed into a sterile plant, a male sterile plant, or a transgenic plant or transgenic double-flowered plant with suppressed anther dihescence.

The following will describe the transcription factor and functional peptide.

### (I-1-a) Transcription Factor for Promoting Transcription of a Gene associated with Formation of Floral Organs

The transcription factor used in the present invention is not particularly limited as long as it can promote transcription of genes associated with formation of floral organs. Such transcription factors are conserved in a wide variety of plants. As such, the transcription factor used in the present invention includes proteins conserved in many plants and having similar functions.

Non-limiting examples of such transcription factors are APETALA3 protein and PISTILLATA protein, which are transcription factors including MADS Box.

APETALA3 protein is a representative example of the transcription factor used in the present invention. The protein, having the amino acid sequence of SEQ ID NO: 134, is known as a transcription factor that promotes transcription of a gene associated with formation of floral organs. In *Arabidopsis thaliana,* a mutant line of a gene that encodes APETALA3 protein (hereinafter referred to as "APETALA3 gene" for convenience of explanation) is known to inhibit formation of petal and stamen (see Thomas Jack, Laura L. Brockman, and Elliot M. Meyerrowitz., Cell, Vol 68, pp 683-697, February, 1992). In the present invention, for example, APETALA3 protein is fused with a functional peptide to be described later, so as to convert the transcription factor, APETALA3 protein, into a transcription repressor.

The transcription factor used in the present invention is not just limited to the APETALA3 protein having the amino acid sequence of SEQ ID NO: 134, as long as it can promote expression of a gene associated with formation of floral organs. Specifically, a protein with the substitution, deletion, insertion, and/or addition of one to several amino acids in the amino acid sequence of SEQ ID NO: 134 can be used in the present invention as long as it has the foregoing function. Referring to the phrase "substitution, deletion, insertion, and/or addition of one to several amino acids," the number of amino acids substituted, deleted, inserted, and/or added in the amino acid sequence of SEQ ID NO: 134 is not particularly limited. For example, the number of amino acids is 1 to 20, preferably 1 to 10, more preferably 1 to 7, further preferably 1 to 5, and particularly preferably 1 to 3.

Concerning the transcription factor that promotes transcription of a gene associated with formation of floral organs, the amino acid sequence of the transcription factor used in the present invention is believed to be highly conserved in plants of many different species. As such, the transcription factor, or its gene, for promoting expression of genes associated with formation of floral organs is not necessarily required to be isolated from each individual plant for which male sterility is desired. That is, a chimeric protein constructed in *Arabidopsis thaliana* can be introduced into other plants as will be described later in Examples, so as to easily produce male sterile plants in a wide range of plant species.

For the production of a chimeric protein used in the present invention, conventional genetic recombination techniques can be suitably used, as will be described later. Thus, a plant producing process according to the present invention can suitably use a gene that encodes the transcription factor.

The gene that encodes the transcription factor is not particularly limited. A specific example is APETALA3 gene when the transcription factor is APETALA3 protein. For example, APETALA3 gene is a polynucleotide that has the base sequence of SEQ ID NO: 135 as an open reading frame (ORF).

The APETALA3 gene used in the present invention, or the coding gene of the transcription factor is not just limited to the foregoing example. For example, a homologue of a gene having the base sequence of SEQ ID NO: 135 may be used. A specific example is a gene that hybridizes under stringent conditions with a gene of a base sequence complementary to the base sequence of SEQ ID NO: 135, and that encodes the transcription factor. Note that, as used herein, "hybridize under stringent conditions" means binding under washing conditions of 2 × SSC at 60°C.

Hybridization can be performed by conventional methods, for example, according to the procedure described in J. Sambrook et al. Molecular Cloning, A Laboratory Manual, 2nd Ed., Cold Spring Harbor Laboratory (1989). As a rule, the level of stringency increases (more difficult to hybridize) with increase in temperature and decrease in salt concentration.

The method by which the coding gene of the transcription factor is obtained is not particularly limited, and the gene can be isolated from a wide variety of plants by conventional methods. For example, a primer pair that has been constructed based on the base sequence of a known transcription factor can be used. With such a primer pair, the gene can be obtained, for example by PCR for example, using the cDNA or genomic DNA of a plant as a template. Alternatively, the coding gene of the transcription factor can be chemically synthesized by conventional methods.

### (I-1-b) Transcription Factor for Promoting Transcription of a Gene associated with Dehiscence of Anther

The transcription factor used in the present invention is not particularly limited as long as it can promote transcription of a gene associated with dehiscence of anther. Generally, the anther dehisces to release the pollen. Thus, the transcription factor that promotes transcription of a gene associated with dehiscence of anther is conserved in many plants. Accordingly, the transcription factor used in the present invention includes functionally redundant transcription factors that are conserved in a variety of plants.

A representative example of the transcription factor used in the present invention is NACAD1 protein. NACAD1 protein is a protein with the amino acid sequence represented by SEQ ID NO: 136, and it belongs to the NAC family proteins of *Arabidopsis thaliana.* In the present invention, for example, the NACAD1 protein is fused with a functional peptide (described later) to convert the transcription factor, NACAD1, into a transcription repressor.

The transcription factor used in the present invention is not just limited to the NACAD1 protein having the amino acid sequence of SEQ ID NO: 136, as long as it can promote transcription of a gene associated with dehiscence of anther. Specifically, a protein with the substitution, deletion, insertion, and/or addition of one to several amino acids in the amino acid sequence of SEQ ID NO: 136 can be used in the present invention as long as it has the foregoing function. Referring to the phrase "substitution, deletion, insertion, and/or addition of one to several amino acids," the number of amino acids substituted, deleted, inserted, and/or added in the amino acid sequence of SEQ ID NO: 136 is not particularly limited. For example, the number of amino acids is 1 to 20, preferably 1 to 10, more preferably 1 to 7, further preferably 1 to 5, and particularly preferably 1 to 3.

The transcription factor also includes a protein sharing not less than 20%, preferably not less than 50%, or more preferably not less than 60% or 70% homology with the amino acid sequence of SEQ ID NO: 136, and that is capable of promoting transcription of a gene associated with dehiscence of anther. As used herein, the term "homology" refers to the proportion of the same amino acid sequence. The higher the homology, the closer the relationship between the two. For example, the transcription factor may be a NAC factor sharing 52% homology and having the same function as the NACAD1 protein having the amino acid sequence of SEQ ID NO: 136.

For the production of a chimeric protein used in the present invention, conventional genetic recombination techniques can be suitably used, as will be described later. Thus, a plant producing process according to the present invention can suitably use a gene that encodes the transcription factor.

The gene that encodes the transcription factor is not particularly limited as long as it corresponds to the amino acid sequence of the transcription factor based on the genetic code. When the transcription factor is NACAD1 protein, a specific example is a gene that encodes NACAD1 protein (hereinafter referred to as "NACAD1 gene" for convenience of explanation). A specific example of NACAD1 gene is a polynucleotide that has the base sequence of SEQ ID NO: 137 as an open reading frame (ORF).

The NACAD1 gene used in the present invention, or the coding gene of the transcription factor is not just limited to the foregoing example. For example, a homologue of a gene having the base sequence of SEQ ID NO: 137 may be used. A specific example is a gene that hybridizes under stringent conditions with a gene of a base sequence complementary to the base sequence of SEQ ID NO: 137, and that encodes the transcription factor. Note that, as used herein, "hybridize under stringent conditions" has the meaning as defined above.

As described earlier, hybridization can be performed by conventional methods. Further, as described above, the method by which the coding gene of the transcription factor is obtained is not particularly limited, and the gene can be isolated from a wide variety of plants by conventional methods. Alternatively, the coding gene of the transcription factor can be chemically synthesized by conventional methods, as mentioned above.

### (1-1-c) MYB Domain-Containing Transcription Factor for Promoting Transcription of a Gene Associated with Dehiscence of Anther

The transcription factor used in the present invention is not particularly limited as long as it can promote transcription of a gene associated with dehiscence of anther and contains the MYB domain. As used herein, the MYB domain refers to a unit domain of about 50 amino acid residues that are homologous to the product of myb gene, one type of cancer gene. Transcription factors with the MYB domain are members of the MYB transcription factor family, and the MYB domain is conserved in a wide variety of plant and animal species. The transcription factor used in the present invention is not particularly limited as long as it belongs to the MYB transcription factor family and is capable of promoting transcription of a gene associated with dehiscence of anther. Generally, the anther dehisces and releases pollen. Thus, the transcription factor used in the present invention includes functionally redundant and MYB domain-containing transcription factors that are conserved in a variety of plants.

Non-limiting examples of such transcription factors include *Arabidopsis thaliana* MYB26 protein, and a protein encoded by rice NP_916576.1 (GenBank accession number).

The *Arabidopsis thaliana* MYB26 protein is a representative example of the transcription factor used in the present invention. The MYB26 protein may have the amino acid sequence of SEQ ID NO: 138 for example, and, as mentioned above, it is a member of the *Arabidopsis thaliana* MYB transcription factor family. It is known that variable splicing produces a number of splicing variants in living organisms of higher form in particular. Splicing variants are also found in MYB26 protein. Thus, the MYB26 protein is not just limited to the protein with the amino acid sequence of SEQ ID NO: 138 and it includes the splicing variants, provided that the splicing variants can promote transcription of a gene associated with dehiscence of anther.

In the present invention, for example, MYB26 protein is fused with a functional peptide to be described later, so as to convert the transcription factor, MYB26 protein, into a transcription repressor.

It should be noted here that little is known about the gene associated with dehiscence of anther, i.e., the target genes of MYB26 protein. It is envisaged, however, that the target genes of the transcription factor MYB26 is a gene of, for example, an enzyme involved in the lignin synthesis of anther. More specifically, it is assumed that MYB26 is a transcription factor that positively controls gene expression of an enzyme or the like involved in the lignin synthesis of anther. In one possible model that describes the relationship between lignin synthesis and anther dehiscence, the endothelial cells of the anther wall contract by undergoing lignification and dehydration, and this opens up the anther along the stomium. Thus, if the target genes of the transcription factor used in the present invention are genes of an enzyme or the like involved in lignin synthesis, then it can be said that the transcription factor used in the present invention is the transcription factor that promotes transcription of a gene associated with anther dehiscence, and that positively controls gene expression of the enzyme involved in lignin synthesis.

The transcription factor used in the present invention is not just limited to the MYB26 protein with the amino acid sequence of SEQ ID NO: 138, as long as it is a MYB family transcription factor that promotes transcription of a gene associated with dehiscence of anther. Specifically, a protein with the substitution, deletion, insertion, and/or addition of one to several amino acids in the amino acid sequence of SEQ ID NO: 138 can be used in the present invention as long as it has the foregoing function and the MYB domain. Referring to the phrase "substitution, deletion, insertion, and/or addition of one to several amino acids," the number of amino acids substituted, deleted, inserted, and/or added in the amino acid sequence of SEQ ID NO: 138 is not particularly limited. For example, the number of amino acids is 1 to 20, preferably 1 to 10, more preferably 1 to 7, further preferably 1 to 5, and particularly preferably 1 to 3.

The transcription factor also includes a protein sharing not less than 20%, preferably not less than 50%, or more preferably not less than 60% or 70% homology with the amino acid sequence of SEQ ID NO: 138, capable of promoting transcription of a gene associated with dehiscence of anther, and having the MYB domain. As used herein, the term "homology" refers to the proportion of the same amino acid sequence. The higher the homology, the closer the relationship between the two.

As used herein, the transcription factor for promoting transcription of a gene associated with anther dehiscence and having the MYB domain has an amino acid sequence that is considered to be highly conserved among a wide variety of plants of many different species. Therefore, it is not necessarily required to isolate the transcription factor and a coding gene of the transcription factor in the individual plants in which dehiscence of anther is to be suppressed. That is, as will be described later in Examples, a coding gene of a chimeric protein constructed in *Arabidopsis thaliana* can simply be introduced into other plants, so that plants in which anther dehiscence is suppressed can easily be produced in a wide variety of plants.

For the production of a chimeric protein used in the present invention, conventional genetic recombination techniques can be suitably used, as will be described later. Thus, a plant producing process according to the present invention can suitably use a gene that encodes the transcription factor.

The gene that encodes the transcription factor is not particularly limited as long as it corresponds to the amino acid sequence of the transcription factor based on the genetic code. When the transcription factor is MYB26 protein, a specific example is a gene that encodes MYB26 protein (hereinafter referred to as "MYB26 gene" for convenience of explanation). For example, MYB26 gene is a polynucleotide that has the base sequence of SEQ ID NO: 139 as an open reading frame (ORF).

The MYB26 gene used in the present invention, or the coding gene of the transcription factor is not just limited to the foregoing example. For example, a homologue of a gene having the base sequence of SEQ ID NO: 139 may be used. A specific example is a gene that hybridizes under stringent conditions with a gene of a base sequence complementary to the base sequence of SEQ ID NO: 139, and that encodes the transcription factor. Note that, as used herein, "hybridize under stringent conditions" has the meaning as defined above.

As described earlier, hybridization can be performed by conventional methods. Further, as described above, the method by which the coding gene of the transcription factor is obtained is not particularly limited, and the gene can be isolated from a wide variety of plants by conventional methods. Alternatively, the coding gene of the transcription factor can be chemically synthesized by conventional methods, as mentioned above.

### (I-1-d) Transcription Factor associated with Formation of Stamen and Pistil

The transcription factor used in the present invention is not particularly limited as long as it is a transcription factor associated with formation of stamen and pistil. In one embodiment of the present invention, the transcription factor used in the present invention is a protein with the amino acid sequence of SEQ ID NO: 140, or a mutant protein with the amino acid sequence of SEQ ID NO: 140. The protein with the amino acid sequence of SEQ ID NO: 140 is a transcription factor encoded by the AG gene, and is associated with formation of stamen and pistil.

The mutant includes, for example, deletion, insertion, reversion, recursion, and type substitution (for example, substitution of a hydrophilic residue with a different hydrophilic residue, but it is not common to substitute a strong hydrophilic residue with another strong hydrophilic residue). Generally, substitution of an (neutral) amino acid in a protein rarely affects the protein activity.

It is well known in the art that some of the amino acids in the amino acid sequence of a protein can easily be modified without significantly affecting the structure or function of the protein. It is also known that such a mutant with no significant structural or functional change occurs not only in artificially modified proteins but in nature as well.

It is easy for a person ordinary skill in the art to modify one to several amino acids in the amino acid sequence of a protein using a conventional technique. For example, by a conventional point mutation introducing method, any base of a polynucleotide that encodes a protein can be mutated. Further, with a primer that is designed to correspond to an arbitrary site of a polynucleotide that encodes a protein, a deletion mutant or an addition mutant can be produced. Further, with the method described in the present invention, whether or not the mutant has desired activities can easily be evaluated.

The mutant preferably includes substitution, deletion, or addition of amino acid, which may be conservative or non-conservative. Silent substitution, silent addition, and silent deletion are preferable, and conservative substitution is particularly preferable. Neither of these modifications changes the protein activities as described in the present invention.

Representative examples of conservative substitution include: substitution of one of aliphatic amino acids Ala, Val, Leu, and Ile with another amino acid; exchange of hydroxyl residues Ser and Thr; exchange of acidic residues Asp and Glu; substitution between amide residues Asn and Gln; exchange of basic residues Lys and Arg; and substitution between aromatic residues Phe and Tyr.

For further guidance as to which amino acid change is phenotypically silent (whether the change brings about any adverse effects on the protein activities), refer to Bowie, J.U. et al., Deciphering the Message in Protein Sequence: Tolerance to Amino Acid Substitutions, Science, 247: 1306-1310 (1990) (herein incorporated by reference).

The transcription factor according to the present embodiment is preferably a protein as defined in (a) or (b) below.
- (a): A protein with the amino acid sequence of SEQ ID NO: 140.
- (b): A protein with substitution, deletion, insertion, and/or addition of one to several amino acids in the amino acid sequence of SEQ ID NO: 140.

Referring to the phrase "substitution, deletion, insertion, and/or addition of one to several amino acids in the amino acid sequence of (i)," and "substitution, deletion, insertion, and/or addition of one to several amino acids in the amino acid sequence of SEQ ID NO: 140," the number of amino acids substituted, deleted, inserted, and/or added is not particularly limited. For example, the number of amino acids is 1 to 20, preferably 1 to 10, more preferably 1 to 7, further preferably 1 to 5, and particularly preferably 1 to 3.

Such a mutant protein is not limited to a protein that is artificially mutated by a conventional mutant protein producing method, and it may be obtained by isolating and purifying naturally occurring proteins.

The protein according to the present invention is not particularly limited as long as it is made up of amino acids bonded together by peptide bonding. The protein according to the present invention may be a conjugate protein that includes a non-protein structure. As used herein, the "non-protein structure" refers to a sugar chain or an isoprenoid group, for example. However, the meaning of the term is not particularly limited.

The protein according to the present invention may include an additional protein. An example of an additional protein is an epitope-labeled protein such as His, Myc, or Flag.

The homeotic gene of flowers has been isolated from *Arabidopsis thaliana, Antirrhinum majus,* and other plants, and the function of the homeotic gene is considered to be the same in dicots and monocots. It is therefore believed that the amino acid sequence of the transcription factor or the like that is encoded by the AG gene and is associated with formation of stamen and pistil is highly conserved among a wide variety of plants of many different species. As such, in individual plants used to form double-flowered plants, it is not necessarily required to isolate the transcription factor, or its gene, associated with formation of stamen and pistil. More specifically, a chimeric protein constructed in *Arabidopsis thaliana* can be introduced into other plants as will be described later in Examples, so as to produce double-flowered plants in a wide range of plant species.

For the production of a chimeric protein used in the present invention, conventional genetic recombination techniques can be suitably used, as will be described later. Thus, a plant producing process according to the present invention can suitably use a gene (polynucleotide) that encodes the transcription factor.

A gene that encodes the transcription factor may be in the form of RNA (for example, mRNA) or DNA (for example, cDNA or genomic DNA). The DNA may be double stranded or single stranded. The single strand DNA or RNA may be a coding strand (also known as a sense strand) or a non-coding strand (also known as an anti-sense strand).

Further, the gene that encodes the transcription factor may be a mutant of the gene that encodes the transcription factor associated with formation of stamen and pistil. The mutant may be of naturally occurring form as in naturally occurring allele mutations. The "allele mutation" produces one of the alternate forms of a gene that occupies a predetermined locus on a chromosome. Mutations that do not occur in nature can be produced by a conventional mutation inducing technique known in the art.

For example, the mutant may include deletion, substitution, or addition of one to several bases in the base sequence of a polynucleotide that encodes the transcription factor. The mutant may have a mutation in the coding region or non-coding region, or both of these regions. The mutation in the coding region may be deletion, substitution, or addition of amino acid, which may be conservative or non-conservative.

A gene that encodes the transcription factor includes (i) a gene that encodes the transcription factor and (ii) a polynucleotide that hybridizes with the gene under stringent hybridization conditions.

In the present embodiment, a gene that encodes the transcription factor is preferably a polynucleotide as defined in (c) or (d) below.
- (c): A gene that has the base sequence of SEQ ID NO: 141 as an open reading frame.
- (d): A gene that hybridizes under stringent conditions with a gene of a base sequence complementary to the base sequence of SEQ ID NO: 141, and that encodes the transcription factor associated with formation of stamen and pistil.

As used herein, "under stringent conditions" means that hybridization occurs only when the sequences share at least 90% homology, preferably at least 95% homology, or more preferably at least 97% homology.

Hybridization can be performed by conventional methods. As a rule, the level of stringency increases (more difficult to hybridize) with increase in temperature and decrease in salt concentration. Thus, with the increased level of stringency, more homologous polynucleotides can be obtained. Hybridization can suitably be performed under conventional conditions. Though not limited to the following, hybridization can be performed under the following conditions, for example: 42°C, 6 × SSPE, 50% formamide, 1% SDS, 100 µg/ml salmon sperm DNA, 5 × Denhart solution (1 × SSPE; 0.18 M sodium chloride, 10 mM sodium phosphate, pH 7.7, 1 mM EDTA. 5 × Denhart solution; 0.1% bovine serum albumin, 0.1 % Ficoll, 0.1% polyvinyl pyrrolidone).

The method by which a gene that encodes the transcription factor is obtained is not particularly limited. In one method, a DNA fragment that includes a polynucleotide that encodes the transcription factor is isolated and cloned according to conventional techniques. For example, a probe is prepared that specifically hybridizes with a portion of the base sequence of a gene that encodes the transcription factor, and a genomic DNA library or cDNA library is screened with the probe. The probe may have any sequence and/or any length as long as it can specifically hybridize with at least a portion of the base sequence, or its complementary sequence, of a gene that encodes the transcription factor.

Alternatively, a gene that encodes the transcription factor can be obtained using amplification means such as PCR. For example, primers are prepared from the 5' end and 3' end of the sequence, or its complementary sequence, of the cDNA of a polynucleotide that encodes the transcription factor, and PCR or other amplification methods is performed with the primers, using the genomic DNA (or cDNA) as a template for example. By thus amplifying the DNA region between the primers, DNA fragments containing the transcription factor-encoding polynucleotide can be obtained in mass quantity.

### (I-2) Functional Peptide for Converting an arbitrary Transcription Factor into a Transcription Repressor

A functional peptide for converting an arbitrary transcription factor into a transcription factor repressor (will be referred to as "transcription repressor converting peptide" for convenience of explanation) is not particularly limited, as long as it can form a chimeric protein with the transcription factor and thereby suppress transcription of target genes controlled by the transcription factor. Specifically, the transcription repressor converting peptide found by the inventors of the present invention can be used, for example (see, for example, Patent Documents 3 to 9, Non-Patent Documents 10 and 11).

The inventors of the present invention have found that some members of *Arabidopsis thaliana* proteins AtERF3, AtERF4, AtERF7, and AtERF8, which belong to the class II ERF genes, exhibit a notable gene transcription suppressing effect when they are bound to the transcription factor. Base on this finding, the inventors constructed effecter plasmids that include (i) coding genes of these proteins and (ii) DNA excised from these genes. By inserting these effecter plasmids into plant cells, gene transcription was successfully suppressed (see Patent Documents 3 to 6, for example). The same experiment was carried out with genes that encode tobacco ERF3 protein (see Patent Document 7, for example) and rice OsERF3 protein (see Patent Document 8, for example), which belong to the class II ERF genes; and genes that encode *Arabidopsis thaliana* ZAT10 and *Arabidopsis thaliana* ZAT11, which belong to the zinc finger protein genes. Gene transcription was successfully suppressed in all of these experiments. The inventors have also found that these proteins shared a common motif of aspartic acid-leucine-asparagine (DLN) in a C terminus region. A further study of the proteins having such a common motif revealed that the protein that suppresses gene transcription can be a peptide of a very simple structure, and that a peptide of such a simple structure is indeed capable of converting an arbitrary transcription factor into a transcription repressor.

Further, the inventors have also found that *Arabidopsis thaliana* SUPERMAN protein does not share the common motif, yet is capable of converting an arbitrary transcription factor into a transcription repressor. It was also found that a chimeric gene in which a coding gene of the SUPERMAN protein is bound to a DNA binding domain of the transcription factor or a coding gene of the transcription factor serves as a strong transcription repressor.

Thus, in the present embodiment, examples of transcription repressor converting peptides used in the present invention include: class II ERF *Arabidopsis thaliana* proteins such as AtERF3, AtERF4, AtERF7, and AtERF8; tobacco ERF3 protein and rice OsERF3 protein, which are also members of class II ERF proteins; zinc finger proteins such as *Arabidopsis thaliana* ZAT10, *Arabidopsis thaliana* ZAT11, and SUPERMAN protein; peptides excised from these proteins; and synthetic peptides having the foregoing functions.

As to the specific structure of the transcription repressor converting peptide, the transcription repressor converting peptide has the amino acid sequence as defined by any one of the following Formulae (1) through (4), for example.
(1) X1-Leu-Asp-Leu-X2-Leu-X3
   (where X1 represents 0 to 10 amino acid residues, X2 represents Asn or Glu, and X3 represents at least six amino acid residues)
(2) Y1-Phe-Asp-Leu-Asn-Y2-Y3
   (where Y1 represents 0 to 10 amino acid residues, Y2 represents Phe or Ile, and Y3 represents at least six amino acid residues)
(3) Z1-Asp-Leu-Z2-Leu-Arg-Leu-Z3
   (where Z1 represents Leu, Asp-Leu, or Lue-Asp-Leu, Z2 Glu, Gln or Asp, and Z3 represents 0 to 10 amino acid residues.
(4) Asp-Leu-Z4-Leu-Arg-Leu
   (where Z4 represents Glu, Gln, or Asp)

### (I-2-1) Transcription Repressor Converting Peptide of Formula (1)

In the transcription repressor converting peptide of Formula (1), X1 represents 0 to 10 amino acid residues. The type of amino acid constituting the amino acid residue represented by X1 is not particularly limited and any amino acid can be used. In other words, the transcription repressor converting peptide of Formula (1) may include one amino acid of any kind or an oligomer of 2 to 10 amino acid residues of any kind attached to the N terminus, or no amino acid may be attached at all.

For ease of synthesis of the transcription repressor converting peptide of Formula (1), the amino acid residues represented by X1 should be as short as possible. Preferably, the number of amino acid residues should be 10 or less, or more preferably 5 or less.

Further, in the transcription repressor converting peptide of Formula (1), X3 represents at least six amino acid residues. The type of amino acid constituting the amino acid residue represented by X3 is not particularly limited and any amino acid can be used. In other words, the transcription repressor converting peptide of Formula (1) may include an oligomer of six or more amino acid residues of any kind attached to the C terminus. The number of amino acid residues represented by X3 needs to be at least six to exhibit the foregoing functions.

In the transcription repressor converting peptide of Formula (1), the pentamer (5 mer) of five amino acid residues, excluding X1 and X3, has the sequence as represented by SEQ ID NO: 41 or 42. The pentamer has the amino acid sequence of SEQ ID NO: 41 when X2 is Asn and the amino acid sequence of SEQ ID NO: 42 when X2 is Glu.

### (I-2-2) Transcription Repressor Converting Peptide of Formula (2)

In the transcription repressor converting peptide of Formula (2), Y1 represents 0 to 10 amino acid residues as does X1 in the transcription repressor converting peptide of Formula (1). The type of amino acid constituting the amino acid residue represented by Y1 is not particularly limited and any amino acid can be used. In other words, the transcription repressor converting peptide of Formula (2) may include one amino acid of any kind or an oligomer of 2 to 10 amino acid residues of any kind attached to the N terminus, or no amino acid may be attached at all.

For ease of synthesis of the transcription repressor converting peptide of Formula (2), the amino acid residues represented by Y1 should be as short as possible. Preferably, the number of amino acid residues should be 10 or less, or more preferably 5 or less.

Further, in the transcription repressor converting peptide of Formula (2), Y3 represents at least six amino acid residues as does X3 in the transcription repressor converting peptide of Formula (1). The type of amino acid constituting the amino acid residue represented by Y3 is not particularly limited and any amino acid can be used. In other words, the transcription repressor converting peptide of Formula (2) may include an oligomer of six or more amino acid residues of any kind attached to the C terminus. The number of amino acid residues represented by Y3 needs to be at least six to exhibit the foregoing functions.

In the transcription repressor converting peptide of Formula (2), the pentamer (5 mer) of five amino acid residues, excluding Y1 and Y3, has the sequence as represented by SEQ ID NO: 43 or 44. The pentamer has the amino acid sequence of SEQ ID NO: 43 when Y2 is Phe and the amino acid sequence of SEQ ID NO: 44 when Y2 is Ile. The tetramer (4 mer) of four amino acid residues, excluding Y2, has the sequence represented by SEQ ID NO: 45.

### (I-2-3) Transcription Repressor Converting Peptide of Formula (3)

In the transcription repressor converting peptide of Formula (3), the amino acid residues represented by Z1 include 1 to 3 amino acids, including Leu. Z1 is Leu when the number of amino acids is one, Asp-Leu when the number of amino acids is two, and Lue-Asp-Leu when the number of amino acids is three.

In the transcription repressor converting peptide of Formula (3), Z3 represents 0 to 10 amino acid residues, as does X1 in the transcription repressor converting peptide of Formula (1) for example. The type of amino acid constituting the amino acid residue represented by Z3 is not particularly limited and any amino acid can be used. In other words, the transcription repressor converting peptide of Formula (3) may include one amino acid of any kind or an oligomer of 2 to 10 amino acid residues of any kind attached to the C terminus, or no amino acid may be attached at all.

For ease of synthesis of the transcription repressor converting peptide of Formula (3), the amino acid residues represented by Z3 should be as short as possible. Preferably, the number of amino acid residues should be 10 or less, or more preferably 5 or less. Non-limiting examples of amino acid residues represented by Z3 include Gly, Gly-Phe-Phe, Gly-Phe-Ala, Gly-Tyr-Tyr, and Ala-Ala-Ala.

The total number of amino acid residues in the transcription repressor converting peptide of Formula (3) is not particularly limited. However, for ease of synthesis, the total number of amino acid residues is preferably 20 or less.

In the transcription repressor converting peptide of Formula (3), the oligomer (7 mer) of five amino acid residues, excluding Z3, has the sequence as represented by SEQ ID NO: 46 to 54. The oligomer has the amino acid sequence of SEQ ID NO: 46, 47, or 48 when Z1 is Leu and Z2 is Glu, Gln, or Asp, respectively. The oligomer has the amino acid sequence of SEQ ID NO: 49, 50, or 51 when Z1 is Asp-Leu and Z2 is Glu, Gln, or Asp, respectively. The oligomer has the amino acid sequence of SEQ ID NO: 52, 53, or 54 when Z1 is Leu-Asp-Leu and Z2 is Glu, Gln, or Asp, respectively.

### (I-2-4) Transcription Repressor Converting Peptide of Formula (4)

The transcription repressor converting peptide of Formula (4) is a hexamer (6 mer) of six amino acid residues, and it has the amino acid sequence of SEQ ID NO: 5, 14, or 55. The hexamer has the amino acid sequence of SEQ ID NO: 5 when Z4 is Glu, the amino acid sequence of SEQ ID NO: 14 when Z4 is Asp, and the amino acid sequence of SEQ ID NO: 55 when Z4 is Gln.

The transcription repressor converting peptide used in the present invention may be a peptide that has, for example, the hexamer of Formula (4) as the smallest sequence. For example, the amino acid sequence of SEQ ID NO: 7 corresponds to the sequence of amino acids 196 to 201 of *Arabidopsis thaliana* SUPERMAN protein (SUP protein), and it is a sequence which the inventors of the present invention have found to be the transcription repressor converting peptide.

### (I-2-5) Specific Examples of Transcription Repressor Converting Peptide

More specific examples of the transcription repressor converting peptides represented by the foregoing Formulae are peptides with the amino acid sequences of SEQ ID NO: 1 through 17. These oligopeptides were found to be the transcription repressor converting peptides by the inventors of the present invention (see Patent Document 9, for example).

Another specific example of the transcription repressor converting peptides is the oligopeptide as defined in (e) or (f) below.
- (e): A peptide with the amino acid sequence of SEQ ID NO: 18 or 19.
- (f): A peptide with the substitution, deletion, insertion, and/or addition of one to several amino acids in the amino acid sequence of SEQ ID NO: 18 or 19.

The peptide with the amino acid sequence of SEQ ID NO: 18 is SUP protein. Referring to the phrase "substitution, deletion, insertion, and/or addition of one to several amino acids," the number of amino acids substituted, deleted, inserted, and/or added in the amino acid sequence of SEQ ID NO: 18 or 19 is not particularly limited. For example, the number of amino acids is 1 to 20, preferably 1 to 10, more preferably 1 to 7, further preferably 1 to 5, and particularly preferably 1 to 3.

The deletion, substitution, or addition of amino acid can be brought about by modifying the base sequence that encodes the peptide, using a method known in the art. The base sequence can be mutated by a conventional method, such as the Kunkel method or Gapped duplex method, or methods according to these techniques. For example, mutation can be introduced with a mutation introducing kit employing site-specific mutagenesis (for example, Mutant-K or Mutant-G, commercially available from TAKARA), or the LA PCR in vitro Mutagenesis series kit (commercially available from TAKARA).

The functional peptide is not just limited to a peptide with the total length sequence of the amino acid sequence represented by SEQ ID NO: 18, and it may be a peptide with a partial sequence of the amino acid sequence of SEQ ID NO: 18.

An example of such a peptide with a partial sequence is a peptide with the amino acid sequence (amino acid 175 to 204 of the amino acid sequence of SUP protein) represented by SEQ ID NO: 19. Another example is the peptide represented by Formula (3).

### (I-3) Other Examples of Transcription Repressor Converting Peptides

Upon further study on the motif structure, the inventors of the present invention found a motif of six amino acids. The motif is a peptide with the amino acid sequence as represented by the following General Formula (5). This is also a transcription repressor converting peptide.
(5) α1-Leu-β1-Leu-γ1-Leu
   where α1 is Asp, Gln, Asn, Glu, Gln, Thr, or Ser, β1 is Asp, Gln, Asn, Arg, Glu, Thr, Ser, or His, and γ1 is Arg, Gln, Asn, Thr, Ser, His, Lys, or Asp.
   For convenience of explanation, the peptide represented by General Formula (5) is classified into peptides of amino acid sequences represented by the following General Formulae (6) through (9).
(6) α1-Leu-β1-Leu-γ2-Leu
(7) α1-Leu-β2-Leu-Arg-Leu
(8) α2-Leu-β1-Leu-Arg-Leu
(9) Asp-Leu-β3-Leu-Arg-Leu
   where α1 is Asp, Asn, Glu, Gln, Thr, or Ser, α2 is Asn, Glu, Gln, Thr, or Ser, β1 is Asp, Gln, Asn, Arg, Glu, Thr, Ser, or His, β2 is Asn, Arg, Thr, Ser, or His, β3 is Glu, Asp, or Gln, and γ2 is Gln, Asn, Thr, Ser, His, Lys, or Asp.

A more specific example of the transcription repressor converting peptides with the amino acid sequences of SEQ ID NO: (5) to (9) is a peptide with the amino acid sequence represented by SEQ ID NO: 20, 21, 22, 23, 24, 25, 26, 27, 28, 29, 30, 31, 32, 33, 34, 35, 38, 39, 40, or 152. Among these peptides, the peptide of SEQ ID NO: 27, 28, 30, 32, 38, 39, 40, or 152 corresponds to the peptide represented by General Formula (6). The peptide of SEQ ID NO: 20, 23, 33, 34, or 35 corresponds to the peptide represented by General Formula (7). The peptide of SEQ ID NO: 24, 25, 26, 29, or 31 corresponds to the peptide represented by General Formula (8). The peptide of SEQ ID NO: 21 or 22 corresponds to the peptide represented by General Formula (9).

Other than the peptides of General Formulae (5) through (9), a transcription repressor converting peptide with the amino acid sequence represented by SEQ ID NO: 36 or 37 may be used as well.

### (I-4) Producing Process of Chimeric Protein

The transcription repressor converting peptides described in Sections (I-2) and (I-3) may be fused with the transcription factor described in Section (I-1) above. By thus forming a chimeric protein, the transcription factor can be converted into a transcription repressor. Thus, in the present invention, a polynucleotide that encodes the transcription repressor converting peptide is used to obtain a chimeric gene that also includes a polynucleotide that encodes the transcription factor. In this way, the chimeric protein can be produced.

Specifically, a polynucleotide that encodes the transcription repressor converting peptide (will be referred to as "transcription repressor converting polynucleotide" for convenience of explanation) is ligated to a polynucleotide that encodes the transcription factor. A resulting chimeric gene is then introduced into plant cells to produce a chimeric protein. As to the specific method of introducing the chimeric gene into plant cells, detailed explanation will be made later in Section (II).

The base sequence of the transcription repressor converting polynucleotide is not particularly limited as long as it includes a base sequence that, based on the genetic code, corresponds to the amino acid sequence of the transcription repressor converting peptide. Further, as required, the transcription repressor converting polynucleotide may include a base sequence that serves as a ligation site for the polynucleotide that encodes the transcription factor. Further, in the case where there is no registration of reading frames between the transcription repressor converting polynucleotide and the polynucleotide that encodes the transcription factor, the transcription repressor converting polynucleotide may additionally include a base sequence for registering the reading frames.

A specific example of the transcription repressor converting polynucleotide is a polynucleotide of the base sequence represented by SEQ ID NO: 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 122, 124, 126, 128, 130, 132, or 153. These polynucleotides are complementary to the polynucleotides represented by SEQ ID NO: 57, 59, 61, 63, 65, 67, 69, 71, 73, 75, 77, 79, 81, 83, 85, 87, 89, 93, 95, 97, 99, 101, 103, 105, 107, 109, 111, 113, 115, 117, 119, 121, 123, 125, 127, 129, 131, 133, and 154, respectively. Another example of the transcription repressor converting polynucleotide is a polynucleotide represented by SEQ ID NO: 90 or 91. These polynucleotides correspond to the amino acid sequences of SEQ ID NO: 1 to 40, and 152, as shown in Table 1 below.

**[Table 1]**

| Amino Acid Sequence | Base Sequence | Amino Acid Sequence | Base Sequence |
|---|---|---|---|
| SEQ ID NO:1 | SEQ ID NO:56, 57 | SEQ ID NO:22 | SEQ ID NO:96, 97 |
| SEQ ID NO:2 | SEQ ID NO:58, 59 | SEQ ID NO:23 | SEQ ID NO:98, 99 |
| SEQ ID NO:3 | SEQ ID NO:60, 61 | SEQ ID NO:24 | SEQ ID NO:100, 101 |
| SEQ ID NO:4 | SEQ ID NO:62, 63 | SEQ ID NO:25 | SEQ ID NO:102, 103 |
| SEQ ID NO:5 | SEQ ID NO:64, 65 | SEQ ID NO:26 | SEQ ID NO:104, 105 |
| SEQ ID NO:6 | SEQ ID NO:66, 67 | SEQ ID NO:27 | SEQ ID NO:106, 107 |
| SEQ ID NO:7 | SEQ ID NO:68, 69 | SEQ ID NO:28 | SEQ ID NO:108, 109 |
| SEQ ID NO:8 | SEQ ID NO:70, 71 | SEQ ID NO:29 | SEQ ID NO:110, 111 |
| SEQ ID NO:9 | SEQ ID NO:72, 73 | SEQ ID NO:30 | SEQ ID NO:112, 113 |
| SEQ ID NO:10 | SEQ ID NO:74, 75 | SEQ ID NO:31 | SEQ ID NO:114, 115 |
| SEQ ID NO:11 | SEQ ID NO:76, 77 | SEQ ID NO:32 | SEQ ID NO: 116, 117 |
| SEQ ID NO:12 | SEQ ID NO:78, 79 | SEQ ID NO:33 | SEQ ID NO:118, 119 |
| SEQ ID NO:13 | SEQ ID NO:80, 81 | SEQ ID NO:34 | SEQ ID NO:120, 121 |
| SEQ ID NO:14 | SEQ ID NO:82, 83 | SEQ ID NO:35 | SEQ ID NO: 122, 123 |
| SEQ ID NO:15 | SEQ ID NO:84, 85 | SEQ ID NO:36 | SEQ ID NO:124, 125 |
| SEQ ID NO:16 | SEQ ID NO:86, 87 | SEQ ID NO:37 | SEQ ID NO:126, 127 |
| SEQ ID NO:17 | SEQ ID NO:88, 89 | SEQ ID NO:38 | SEQ ID NO:128, 129 |
| SEQ ID NO:18 | SEQ ID NO:90 | SEQ ID NO:39 | SEQ ID NO:130, 131 |
| SEQ ID NO:19 | SEQ ID NO:91 | SEQ ID NO:40 | SEQ ID NO:132, 133 |
| SEQ ID NO:20 | SEQ ID NO:92, 93 | SEQ ID NO:152 | SEQ ID NO:153, 154 |
| SEQ ID NO:21 | SEQ ID NO:94, 95 | | |

A chimeric protein used in the present invention can be obtained from the chimeric gene in which the coding gene of the transcription factor is ligated to the transcription repressor converting polynucleotide. As such, the structure of the chimeric protein is not particularly limited as long as it includes a transcription factor site and a transcription repressor converting peptide site. For example, the chimeric protein may include various types of additional polypeptides, such as a polypeptide for linking the transcription factor and the transcription repressor converting peptide, and a polypeptide, such as His, Myc, or Flag, for epitope-labeling the chimeric protein. Further, the chimeric protein may optionally include a non-polypeptide structure, for example, such as a sugar chain and an isoprenoid group.

### (II) Exemplary Plant Producing Process according to the Present Invention

A plant producing process according to the present invention is not particularly limited as long as it includes the step of producing the chimeric protein of Section (I) in a plant and suppressing expression of a gene associated with formation of floral organs; the step of suppressing dehiscence of anther; or the step of producing a double-flowered plant. More specifically, a plant producing process according to the present invention may include, for example, an expression vector constructing step, a transforming step, and a screening step. The present invention needs to include at least the transforming step. The following describes each step in detail.

### (II-1) Expression Vector Constructing Step

The expression vector constructing step performed in the present invention is not particularly limited as long as it includes the step of constructing a recombinant expression vector that includes: a coding gene of the transcription factor described in Section (I-1); the transcription repressor converting polynucleotide described in Section (I-4); and a promoter.

As the carrier vector of the recombinant expression vector, various types of conventional vectors can be used. Some of the examples include a plasmid, a phage, and a cosmid, which are suitably selected according to the type of plant cell or introducing method. More specific examples are pBR322, pBR325, pUC19, pUC119, pBluescript, pBluescriptSK, and vectors of the pBI family. Binary vectors of the pBI family are preferable when the Agrobacterium method is used to introduce the vector into a plant. Specific examples of pBI binary vectors include pBIG, pBIN19, pBI101, pBI121, and pBI221.

The promoter is not particularly limited as long as it can express the gene in plants, and conventional promoters can be suitably used. Examples of the promoter include cauliflower mosaic virus 35S promoter (CaMV35S), actin promoter, a promoter for nopaline synthetase, tobacco PR1a gene promoter, and a promoter with small subunits of tomato ribulose-1,5-biphosphate carboxylase/oxydase. Among these examples, cauliflower mosaic virus 35S promoter and actin promoter can be suitably used. With these promoters, the resulting recombinant expression vector inserted in plant cells can strongly express the gene of interest. Further, it is preferable that the promoter be able to induce anther-specific gene expression. Examples of such promoters include TA56 promoter, AtMYB26 promoter, and DAD1 promoter. With these promoters, the coding gene of the chimeric protein can be expressed only in anther and dehiscence of anther can be suppressed without affecting other tissues. It is particularly preferable that the promoter be a promoter of a gene that encodes NACAD 1 or similar transcription factors conserved in plants. With such promoter, the gene can be expressed at a specific time and in a specific tissue, making it possible to more effectively suppress dehiscence of anther.

The promoter is not particularly limited as long as (i) it is ligated to express a chimeric gene in which a coding gene of the transcription factor is ligated to the transcription repressor converting polynucleotide, and (ii) it is introduced in the vector. That is, the promoter, as a recombinant expression vector, is not particularly limited to a specific structure.

In addition to the promoter and the chimeric gene, the recombinant expression vector may include other DNA segments. Examples of other DNA segments include, but are not particularly limited to, terminators, selection markers, enhancers, and base sequences for improving translation efficiency. Further, the recombinant expression vector may additionally include a T-DNA region. With the T-DNA region, the efficiency of gene transfer can be improved, particularly when Agrobacterium is used to introduce the recombinant expression vector into plants.

The terminator is not particularly limited and conventional terminators can be used as long as it serves as a transcription termination site. Specifically, a transcription termination region (Nos terminator) of a nopaline synthetase gene, and a transcription termination region of cauliflower mosaic virus 35S (CaMV35S terminator) can be suitable used, for example. The Nos terminator is particularly preferable.

With the terminator suitably placed in the transformation vector, the transformation vector introduced into the plant cell does not cause syntheses of unnecessarily long transcripts, or there will be no reduction in the number of plasmid copies in the presence of a strong promoter.

As the selection marker, a chemical-resistant gene can be used, for example. Specific examples of chemical-resistant genes are those resistant to hygromycin, bleomycin, kanamycin, gentamicin, and chloramphenicol. With such chemical-resistant genes, plants glowing in an antibiotic culture medium can easily be screened for transformants.

As the base sequence for improving translation efficiency, a tobacco mosaic virus omega sequence can be used, for example. With the omega sequence placed in an untranslated region (5' UTR) of the promoter, the translation efficiency of the chimeric gene can be improved. As described so far, the transformant vector may contain various types of DNA segments depending on its purpose.

The method of constructing the recombinant expression vector is not particularly limited. The promoter, the coding gene of the transcription factor, the transcription repressor converting polynucleotide, and optional other DNA segments are introduced in a predetermined order into a suitably selected carrier vector. For example, the coding gene of the transcription factor is ligated to the transcription repressor converting polynucleotide to construct a chimeric gene. The chimeric gene is then ligated to a promoter (and optionally a terminator, etc.) to construct an expression cassette that is inserted into a vector.

In constructing the chimeric gene and the expression vector, the order of DNA segments can be regulated by, for example, having complementary ends at the excision sites of each DNA segment, and then by causing the reaction with a ligase. In the case where the expression cassette contains a terminator, the DNA segments are ordered such that the promoter, the chimeric gene, and the terminator are placed in this order from the upstream side. Further, the type of chemical used to construct the recombinant expression vector is not particularly limited. That is, the type of restriction enzyme or ligase is not particularly limited and may be suitably selected from commercially available products.

Further, the proliferation method (producing method) of the recombinant expression vector is not particularly limited and conventional methods can be used. Generally, *E. coli* is used as a host, and the recombinant expression vector is grown therein. In this case, the type of *E. coli* may be suitably selected according to the type of vector used.

### (II-2) Transforming Step

In a transforming step performed in the present invention, the recombinant expression vector described in Section (II-1) is introduced into plant cells to produce the chimeric protein described in Section (I) above.

The method by which the recombinant expression vector is introduced into plant cells (transforming method) is not particularly limited, and conventional method can be suitably used according to the type of plant cell. Specifically, a method using Agrobacterium, or a method by which the recombinant expression vector is directly introduced in a plant cell can be used. As an example of a method using Agrobacterium, *Transformation of Arabidopsis thaliana by vacuum infiltration* (http://www.bch.msu.edu/pamgreen/protocol.htm) is available.

As examples of a method by which the recombinant expression vector is directly introduced into plant cells, the following methods are available: a micro injection method, an electroporation method, a polyethylene glycol method, a particle gun method, a protoplast fusion method, and a calcium phosphate method.

The plant cell to which the recombinant expression vector is introduced may be, for example, a cell, a callus, or a suspension culture cell of various tissues of plant organs such as a flower, a leaf, and a root.

In a plant producing process according to the present invention, the recombinant expression vector may be suitably selected according to the type of plant to be produced. Alternatively, a multi-purpose recombinant expression vector may be constructed in advance and introduced into plant cells. That is, a plant producing process according to the present invention may or may not contain the recombinant expression vector constructing step described in Section (I-1) above.

### (II-3) Other Steps, Other Methods

A plant producing process according to the present invention includes the transforming step, and may include the recombinant expression vector constructing step. Further, the process may include other steps. For example, a screening step of screening transformed plants for suitable individuals may be included.

The screening method is not particularly limited. For example, the transformants may be screened based on chemical resistance such as hygromycin resistance, or screening may be performed based on the ability of the transformed plants to properly form pollens after they are grown. Further, screening may be made based on a state of anther dehiscence in grown transformants. In this case, for example, the shape of anther may be observed with an electron microscope, a stereo microscope, or the like (see Examples).

Further, screening may be made based on the morphology of the flowers of grown transformants. In this case, for example, morphological comparisons may be made between transformant flowers and non-transformant flowers (see Examples). The flower morphology allows for screening by simple mean of comparison. Further, by comparing plant morphology, the very effect of the present invention, i.e., production of double-flowered plants, can be confirmed.

In a plant producing process according to the present invention, the chimeric gene is introduced into plants and therefore expression of a gene associated with formation of floral organs can also be suppressed in the offspring that is produced either sexually or asexually by the plants. Further, anther dehiscence can also be suppressed in the offspring. Further, double-flowered plants can also be obtained in the offspring. Further, the plant can be mass produced from the cells, seeds, fruits, strains, calluses, stem tubers, cuttings, tubers, or other reproductive parts obtained from the plant or its offspring. Thus, a plant producing process according to the present invention may include a breeding step (mass producing step) of breeding the selected plants.

As used herein, the term "plants" includes any one of adult plants, plant cells, plant tissues, calluses, and seeds. In other words, in the present invention, anything that can be grown into an adult plant is regarded as a plant. The "plant cells" include various types of plant cells, examples of which include suspension culture cells, protoplasts, and a leaf slice. The plants can be obtained by growing and differentiating the plant cells. The plants can be reproduced from the plant cells by a conventional method, which is selected according to the type of plant cell used. Thus, a plant producing process according to the present invention may include reproducing step of reproducing plants from plant cells.

A plant producing process according to the present invention is not just limited to the method that employs transformation with the recombinant expression vector. Specifically, the chimeric protein itself may be administered to plants. In this case, the chimeric protein is administered to plants of an early stage so that expression of a gene associated with formation of floral organs can be suppressed and therefore anther dehiscence can be suppressed or double followers can be obtained in portions of plants to be actually used. The method of administering the chimeric protein is not particularly limited and various conventional methods can be used.

### (III) Plants obtained by the Present Invention, Usefulness and Use of the Plants

In a plant producing process according to the present invention, a coding gene of the chimeric protein is expressed in plants. The transcription factor-derived DNA binding domain of the chimeric protein binds to target genes that is possibly associated with formation of floral organs. Here, the possible target genes associated with formation of floral organs may be target genes that are possibly associated with formation of stamen or pistil. Further, the possible target genes associated with formation of stamen or pistil may be genes that are possibly associated with dehiscence of anther. Further, the possible target genes may be genes that are possibly associated with formation of stamen or pistil.

The transcription factor is converted into a transcription repressor, with the result that transcription of target genes is suppressed. This brings about mutation in the formation of floral organs, and sterile plants are obtained. As a result, dehiscence of anther can be suppressed, or double-flowered plants can be obtained. Thus, the present invention also includes plants that are produced by a plant producing process of the present invention.

As used herein, the "sterile plants" include not only completely sterile plants lacking stamen and pistil but also incompatible plants, which have stamen and pistil yet cannot form seeds. Further, the "sterile plants" include plants which have incomplete stamen-like organ and/or pistil-like organ yet cannot form seeds. Further, for example, the "sterile plants" include male sterile plants, including those in which (i) formation of stamen is inhibited and no pollen is formed at all, (ii) stamen is formed but no pollen is formed due to the lack of anther, (iii) stamen and pistil are formed but the amount of pollen is not enough to cause dehiscence of anther, and (iv) pollens are swollen and fused together, so that there is no spreading of pollens.

By transforming a target plant with the chimeric gene that encodes the chimeric protein, a sterile plant can be obtained very easily without using complicated genetic recombinant techniques. Since sterile plants cannot form seeds, and completely sterile plants and male sterile plants do not deliver pollens, the problems concerning spreading of genetically modified plants in the environment can be prevented.

Male sterile plants cannot self pollinate, but can be crossed with plants of different varieties. Thus, male sterile plants can be suitably used for crosses that take advantage of heterosis, and cultivation of first filial generation with superior traits can be efficiently carried out.

Further, since male sterility always produces hybrids, it is not required to remove the pistil for the cross. This greatly reduces labor. Male sterility is therefore suited for improving efficiency of cross experiments. (III-1) Specific Examples of Plants according to the Present Invention

Sterile plants according to the present invention are not particularly limited to specific kinds, as long as usefulness of the plants can be improved by the acquired sterility, suppressed anther dehiscence, and double flower morphology. The plants may be angiosperms or gymnosperms. Examples of gymnosperms include *Taxodiaceae* plants of order *Taxodiales, Pinaceae* plants, *Cupressaceae* plants, and *Podocarpus* plants. The gymnosperms may be monocots or dicots. Examples of dicots are Brassicaceae plants such as *Arabidopsis thaliana,* and *Theaceae* plants.

The dicots may be *Archichlamiidae* or *Sympetalidae.* Examples of *Sympetalidae* include *Gentianales, Solanales, Lamiales, Callitrichales, Plantaginales, Campanulales, Scrophulariales, Rubiales, Dipsacales,* and *Asterales.* Examples of *Archichlamiidae* include *Dilleniales, Theales, Malvales, Lecythidales, Nepenthales, Violales, Salicales, Capparales, Ericales, Diapensiales, Ebenales,* and *Primulales.* The monocots may be *Poaceae* plants such as rice, corn, and barley (wheat), and *Eriocaulaceae* plants.

Further, sterile plants according to the present invention may be plants whose fruits and seeds have commercial values, or house plants (flower plants) which themselves have commercial values. Thus, more specific examples of sterile plants according to the present invention include: food plants such as rapeseeds, potatoes, marsh grass, soy beans, cabbage, lettuce, tomatoes, cauliflower, French beans, turnips, radish, broccoli, melon, orange, water melon, green onion, and burdock, and house plants such as rose, *Dahlia,* hydrangea, and carnation.

### (III-2) Usefulness of the Present Invention

The present invention is useful in areas where production of the sterile plants has certain values. The following will describe some of the specific examples; however, usefulness of the present invention is not just limited to the examples described below.

With the technique of the present invention, male sterile plants can be produced that cannot properly form pollen, and the male sterile plants can be used for the variety improvement using crosses that take advantage of heterosis. Since pollens cannot be formed properly in the male sterile plants of the present invention, there will be no self-pollination even if the male sterile plants are self-fertilizing plants such as rice. This allows for interbreeding by pollinating the plants with the pollens of other species. As a result, selection can be made, both easily and efficiently, for the heterotic first filial generation of superior traits.

Further, with the technique of the present invention, plants can be produced in which dehiscence of anther is suppressed. The plants can then be used for the variety improvement using crosses that take advantage of heterosis. Since pollens are not released from the anther in the plants in which dehiscence of anther is suppressed, there will be no self-pollination even if the plants are self-fertilizing plants such as rice. This allows for interbreeding by pollinating the plants with the pollens of other species. As a result, selection can be made, both easily and efficiently, for the heterotic first filial generation of superior traits.

The technique of the present invention is also applicable to allogamous plants such as corn. In the variety improvement of allogamous plants currently in practice, the plants are pollinated with the pollens of other species, while avoiding self-pollination by cutting off pistils by hand (male sterilization). There will be no such labor when the technique of the present invention is used to produce male sterile plants or plants in which dehiscence of anther is suppressed. As a result, the time and cost of variety improvement, and the labor required for the cultivation of superior varieties can be greatly reduced.

More specifically, the technique of the present invention produces plants in which fertile pollens are produced and dehiscence of anther is suppressed. The resulting plants are therefore able to produce viable pollens but cannot self-pollinate. This is advantageous for breeding, for example. That is, by producing viable pollens, homozygous individuals can be produced and sustained. By inbreeding these pure lines, a uniform population of seeds can be obtained, with the result that the time and labor required for the screening can be reduced.

The present invention is also applicable to plants, such as onions and potatoes, whose rhizomes have a commercial value. It is known that, in these types of plants, pollination severely inhibits growth of rhizome and reduces the market value of the plants. This is the reason male sterilization is currently required to avoid pollination, and the cost and labor required for the procedure is considerably large. The technique of the present invention produces male sterile plants whose rhizome has a commercial value, or plants in which dehiscence of anther is suppressed. These plants do not require male sterilization, and pollination can be avoided. As a result, the time and cost required to grow plants and produce products can be greatly reduced.

The technique of the present invention is also suitable for plants whose fruits or flowers do not have a commercial value. For example, the technique of the present invention can be used for the prevention of pollen allergy. Specifically, plants that produce a large number of allergy-causing pollen grains can be prevented from releasing pollens if the technique of the present invention were used to produce male sterile plants that cannot properly form pollens. Examples of such allergy-causing plants include: trees such as *Cryptomeria, Chamaecyparis,* and *Chamaecyparis pisifera;* rice plants such as cock's foot grass, Timothy (*Phleum pratense*), and *Poa pratensis;* and weeds such as ragweed, mugwort (*Artemisia princeps Pampan.),* and *Humulus japonicus.* Further, if the technique of the present invention were used to produce plants in which dehiscence of anther is suppressed, then it will be possible to prevent release of pollens from these plants. Thus, if naturally occurring wild type plants were replaced with such male sterile plants or plants in which anther dehiscence is suppressed, then it will be possible to suppress release of allergy-causing pollen grains and thereby prevent pollen allergies.

Further, the technique of the present invention can prevent plant pathology caused by pollen-mediated viral infection. Some types of plant viruses are known to reside in the pollens of sick plants, and transmit to healthy plants through stamen to cause disease. If the technique of the present invention were used to produce a plant that cannot properly form pollen, there will be no pollen-mediated viral infection and no disease will be caused in the plant.

With the technique of the present invention, there will be no spreading of genetically modified plants to nature. For example, eucalyptus, a raw material of pulp, has been genetically modified to introduce superior traits such as salt tolerance, cold resistance, and increased tree size. The introduced traits of these genetically modified plants have been evaluated by conducting experiments in wild environment. However, when grown in wild environment, such genetically modified plants spread in natural environment by the pollen radiation mediated by wind, insects, or other carriers. This may lead to modification of the natural environment. Thus, the evaluation of the genetically modified plants needs to be carried out in a special environment completely isolated from outside.

In this connection, the technique of the present invention is used to transform such genetically modified plants to male sterile plants that cannot form pollen, or plants in which dehiscence of anther is suppressed. In this way, there will be no spreading of the genetically modified plants to nature due to the pollen radiation. This allows the genetically modified plants to be evaluated under conditions that are more similar to wild environment. That is, the introduced traits of the genetically modified traits can be evaluated in a more natural environment.

Further, the technique of the present invention can be used to produce plants in which some of the floral organs, such as the sepal or stamen, are missing or deformed. That is, there is produced a plant with a floral organ of a unique shape not found in conventional flowers. As a result, a novel decorative plant can be produced.

The present invention can produce a double flowered plant. However, usefulness of the present invention is not limited to this particular application, and the invention is applicable to fields in which the production of double flowered plants has certain values. One example is the production of new garden plants.

To describe this particular application more specifically, a plant producing process according to the present invention can be used as follows. First, a gene of a transcription factor is introduced into a cassette vector that has incorporated the functional peptide, and the cassette vector is then introduced into plant cells. With such a simple procedure, the chimeric protein can be expressed in the plant cell, and the transcription of the target genes of the transcription factor can easily be controlled. Further, as described above, since the trait that suppresses the transcription of the target genes of the transcription factor is dominant, the chimeric protein acts dominantly over the transcription factor to suppress transcription of the target genes. It is therefore possible to conveniently and reliably produce double flowered plants in a short time period. This is highly useful in horticulture.

Further, the double flowered plant or sterile plant produced by the present invention does not form seeds. Further, in the completely sterile plant or male sterile plant, the pollen does not spread. Therefore, the genetically recombinant plants do not spread in the environment and are very safe to use.

Further, the double flowered plant or sterile plant produced by the present invention can suitably be used for the heterotic breeding to efficiently produce a first filial generation of superior traits. This is highly useful in agriculture.

### (III-3) Example of Use of the Present Invention

The applicable field or use of the present invention is not particularly limited. For example, the present invention can be used to provide a kit for performing the plant producing process of the present invention, i.e., a sterile plant producing kit.

In one specific example, the sterile plant producing kit at least includes a recombinant expression vector that includes a chimeric gene of (i) a coding gene of the transcription factor and (ii) the transcription repressor converting polynucleotide. More preferably, the sterile plant producing kit includes chemicals for introducing the recombinant expression vector into plant cells. Examples of such chemicals include enzymes and buffers, which are selected according to the type of transformation. Optionally, experimental equipment such as a micro centrifugal tube may be included as well.

### (IV) Producing Process of a Sterile Plant in which Dehiscence of Anther is Controlled

In accomplishing the present invention, the inventors of the present invention found for the first time that NACAD1 and other similar transcription factors conserved in various plants serve as transcription factors that promote transcription of a gene associated with dehiscence of anther. As such, the present invention also includes a producing process of a sterile plant, whereby a coding gene of such a transcription factor is used to control dehiscence of anther.

That is, the present invention provides a producing process of a sterile plant in which dehiscence of anther is controlled, and the process uses a gene that encodes:
(a) a protein with an amino acid sequence represented by SEQ ID NO: 136; or
(b) a protein with the substitution, deletion, insertion, and/or addition of one to several amino acids in the amino acid sequence represented by SEQ ID NO: 136, and capable of promoting transcription of a gene associated with dehiscence of anther,
   or alternatively the process uses:
(c) a gene that has a base sequence of SEQ ID NO: 137 as an open reading frame; or
(d) a gene that hybridizes under stringent conditions with a gene of a base sequence complementary to the gene of the base sequence represented by SEQ ID NO: 137, and that encodes a transcription factor that promotes transcription of a gene associated with dehiscence of anther.

The protein may share no less than 20%, more preferably no less than 50%, or even more preferably no less than 60% or 70% homology with the amino acid sequence of SEQ ID NO: 136, and may be capable of promoting transcription of a gene associated with dehiscence of anther. An example of such a protein is a NAC factor, which shares 52% homology and has the same functions as the NACAD1 protein having the amino acid sequence of SEQ ID NO: 136.

The plant producing process is enabled either by suppressing or over-expressing expression of a gene that encodes the transcription factor associated with dehiscence of anther. Examples of a method that suppresses gene expression includes the anti-sense method, gene targeting method, RNAi method, co-suppression method, and gene disruption tagging method. In one example of a method in which the gene is over-expressed, a vector is constructed that includes a suitable promoter and a gene placed on the downstream side of the promoter, and the vector is introduced into a plant.

The present invention is not limited to the description of the embodiments above, but may be altered by a skilled person within the scope of the claims. An embodiment based on a proper combination of technical means disclosed in different embodiments is encompassed in the technical scope of the present invention.

### [Examples]

Referring to Figure 1(a) through 16, the following will describe the present invention in more detail by way of Examples. It should be noted however that the invention is not limited in any way by the following description.

### [Example 1]

In this Example, a recombinant expression vector was constructed in which a polynucleotide that encodes a 12-amino acid peptide LDLDLELRLGFA (SRDX) (SEQ ID NO: 17), one kind of transcription repressor converting peptide, is ligated to APETALA3 gene and the downstream side of a cauliflower mosaic virus 35S promoter that becomes functional in a plant cell. The recombinant expression vector was then introduced into *Arabidopsis thaliana* by an Agrobacterium method, so as to transform the plant.

### (1) Construction of Plant Transformation vector pBIG2

The plasmid p35S-GFP of Clontech, USA was excised with restriction enzymes HindIII and BamHI, and DNA fragments including the cauliflower mosaic virus 35S promoter were collected after separation by agarose gel electrophoresis.

Plant transformation vector pBIG-HYG provided by Michigan University (Backer, D. 1990 Nucleic Acid Research, 18:203) was excised with restriction enzymes HindIII and SstI, and DNA fragments with no GUS gene were separated by agarose gel electrophoresis.

DNA of the sequences below was synthesized. The DNA was heated at 70°C for 10 minutes, and was allowed to cool and anneal into double strand DNA. The DNA fragment had, from the 5' end, BamHI restriction enzyme site, tobacco mosaic virus omega sequence for improving translation efficiency, restriction enzyme site SmaI, and restriction enzyme sites SalI and SstI.

5'-GATCCACAATTACCAACAACAACAAACAACAAACAACATTACAA
TTACAGATCCCGGGGGTACCGTCGACGAGCT-3' (SEQ ID NO:
159)
5'-CGTCGACGGTACCCCCGGGATCTGTAATTGTAATGTTGTTTGT
TGTTTGTTGTTGTTGGTAATTGTG- (SEQ ID NO: 160)

Then, the DNA fragment with the cauliflower mosaic virus 35S promoter region, and the synthesized double strand DNA were inserted to the HindIII and Sst sites of the pBIG-HYG lacking GUS gene, so as to obtain a plant transformation vector pBIG2.

### (2) Construction of Recombinant Expression Vector pAPETALA3SRDX

### <Isolation of APETALA3 cDNA>

From the cDNA of *Arabidopsis thaliana,* DNA fragments containing only the coding region of APETALA3, except for the end codon, were separated and collected, using the primers below. PCR was carried out in 25 cycles, at a denature temperature of 94°C for 1 minute, an annealing temperature of 47°C for 2 minutes, and an extension temperature of 74°C for 1 minute. In the following, all PCR reactions were performed under the same conditions.
5' primer
5'-GATGGCGAGAGGGAAGATCCAGATCAAG-3' (SEQ IDNO: 161)
3' primer
5'-TTCAAGAAGATGGAAGGTAATGATG-3' (SEQ ID NO:162)

The cDNA of APETALA3 gene, and the amino acid sequence encoded thereby are represented by SEQ ID NO: 135 and 134, respectively.

### <Synthesis of Polynucleotide that Encodes Transcription Repressor Converting Peptide LDLDLELRLGFA (SRDX)>

DNA of the sequences below was synthesized. The DNA was designed so that it encodes 12-amino acid peptide LDLDLELRLGFA (SRDX), and that it has an end codon TAA at the 3' end. The DNA was heated at 70°C for 10 minutes, and was allowed to cool and anneal into double strand DNA.

5'-CTGGATCTGGATCTAGAACTCCGTTTGGGTTTCGCTTAA
G-3' (SEQ ID NO: 163)
5'-CTTAAGCGAAACCCAAACGGAGTTCTAGATCCAGATCCA
G-3' (SEQ ID NO: 164)

### <Construction of Recombinant Expression Vector>

The DNA fragment that includes only the protein coding region of APETALA3 gene, and the DNA fragment that includes the coding region of the transcription repressor converting peptide SRDX were inserted into pBIG2 that had been excised with restriction enzyme SmaI. Constructs that were cloned in the positive direction were isolated to obtain recombinant expression vector p35S::APETALA3SRDX.

### (3) Preparation of Plant Transformed with APETALA3SRDX

Transformation of *Arabidopsis thaliana* with p35S::APETALA3SRDX was carried out according to the procedure described in *Transformation of Arabidopsis thaliana by vacuum infiltration* (http://www.bch.msu.edu/pamgren/protocol.htm). For the infiltration, no vacuum was used. The p35S::APETALA3SRDX was introduced into *Agrobacterium tumefaciens* strain GV3101 (C58C1Rifr) pM90 (Gmr) (koncz and Schell 1986) by an electroporation method. The cells were cultured to OD600 of 1 in 1 liter of YEP medium containing antibodies (kanamycin (Km) 50 µg/ml, gentamicin (Gm) 25 µg/ml, rifampicillin (Rif) 50 µg/ml). The cells were then removed from the culture medium and suspended in 1 liter of infiltration medium (see Table 2 below).

**[Table 2]**

| Infiltration medium (11) | |
|---|---|
| MS salt | 2.29 g |
| Sucrose | 50 g |
| MES to pH5.7 with KOH | 0.5 g |
| benzylaminopurine | 0.044 µM |
| Silwet L-77 | 0.2 ml |

In the solution, 14-day-old *Arabidopsis thaliana* was infiltrated for 1 minute to allow for infection. The plants were then grown again to bear seeds. The seeds were harvested and sterilized for 7 minutes in 50% bleach and 0.02% Triton X-100 solution. After rinsed three times with sterilized water, the seeds were inoculated in sterilized hygromycin selection medium (see Table 3 below).

**[Table 3]**

| Hygromycin Selection Medium | |
|---|---|
| MS salt | 4.3 g/l |
| Sucrose | 1% |
| MES to pH 5.7 with KOH | 0.5 g/l |
| Phytagar | 0.8% |
| Hygromycin | 30 g/ml |
| Vancomycin | 500 ml |

Transformed plants that grew in the hygromycin plate were selected and grown. In this manner, 15 lines of grown plants transformed with p35S::APEALA3SRDX were obtained. Figure 1(a) through Figure 1(c) show an example of the plants.

As shown in Figure 1(a), the plants transformed with p35S::APETALA3SRDX had no petal or stamen in all of the floral organs. The pistils were properly formed.

Further, as shown by the enlarged diagram of Figure 1(b), the plants lacked petal and stamen, and the pistil had its stigma exposed. Further, as shown by the enlarged diagram of Figure 1(c), the plants clearly lacked petal and stamen. Such abnormalities in the floral organs were also observed in the mutant strains of APETALA3 gene. The same result was obtained in all of the 15 lines of transformed plants.

As described above, the plants transformed with p35S::APETALA3SRDX lacked sepal and stamen, and did not properly form pollen. Pollinating the pistil of the transformed plant with the pollens of wild type plants resulted in seed formation. This confirmed that the plants transformed with p35S::APETALA3SRDX were male sterile plants with fertile pistils.

### [Example 2]

In this Example, a recombinant expression vector was constructed in which a polynucleotide that encodes 12-amino acid peptide LDLDLELRLGFA (SRDX) (SEQ ID NO: 17), one kind of transcription repressor converting peptide, is ligated to the downstream side of NACAD1 gene between a cauliflower mosaic virus 35S promoter and the transcription end codon of nopaline synthetase gene. The recombinant expression vector was then introduced into *Arabidopsis thaliana* to transform the plant.

### <Construction of Vector for Constructing Transformation vector>

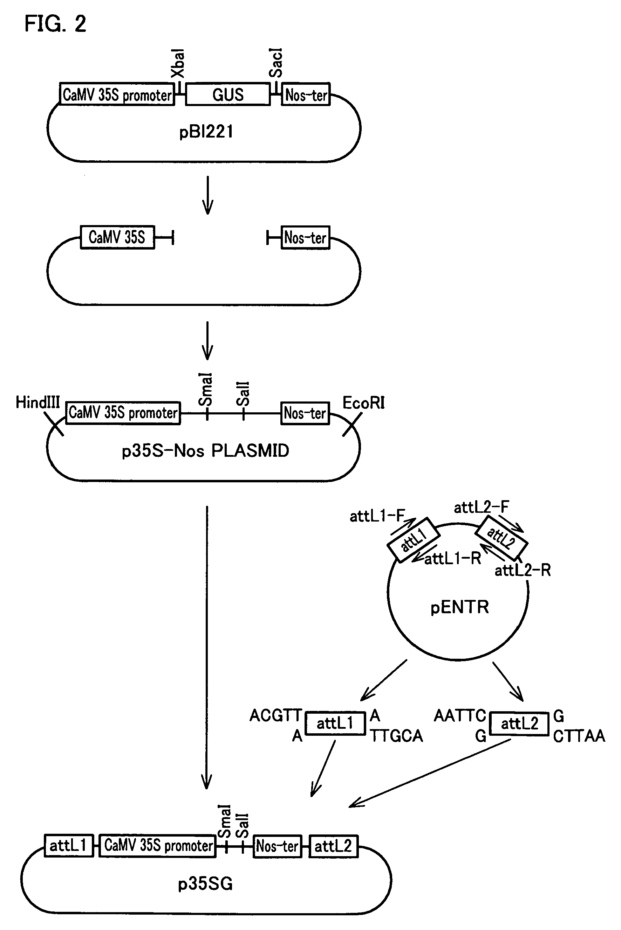

Vector p35SG for constructing a transformation vector was constructed in the following steps (1) through (4), as illustrated in Figure 2.
(1) The attL1 and attL2 regions on the pENTR vector (Invitrogen) were amplified by PCR using primers attL1-F (SEQ ID NO: 142) and attL1-R (SEQ ID NO: 143) for attL1, and primers attL2-F (SEQ ID NO: 144) and attL2-R (SEQ ID NO: 145) for attL2. Resulting attL1 and attL2 fragments were digested with restriction enzymes HindIII and EcoRI, respectively, and were purified. PCR was run under the conditions noted above.
(2) Plasmid pBI221 (Clontech, USA) was excised with restriction enzymes XbaI and SacI, and GUS gene was removed by agarose gel electrophoresis. As a result, 35S-Nos plasmid fragment DNA was obtained that included a cauliflower mosaic 35S promoter (will be referred to as "CaMV35S" hereinafter for convenience of explanation), and a transcription terminator region of nopaline synthetase gene (will be referred to as "Nos-ter" hereinafter for convenience of explanation).
(3) DNA fragments of sequences as represented by SEQ ID NO: 146 and 147 below were synthesized. The DNA fragments were then heated at 90°C for 2 minutes and at 60°C for 1 hour, and were allowed to stand for 2 hours at room temperature (25°C) to anneal into double strand DNA. The DNA fragment was then ligated to the XbaI-SacI region of the 35S-Nos plasmid fragment DNA, so as to obtain p35S-Nos plasmid. The DNA fragments of SEQ ID NO: 146 and 147 each had, at its 5' end, BamHI restriction enzyme site, a tobacco mosaic virus omega sequence for improving translation efficiency, and restriction enzyme sites SmaI, SalI, and SstI. 
5'-ctagaggatccacaattaccaacaacaacaaacaacaaacaacattacaatta
cagatcccgggggtaccgtcgacgagctc-3' (SEQ ID NO: 146)
5'-cgtcgacggtacccccgggatctgtaattgtaatgttgtttgttgtttgttgttgttgg
taattgtggatcct-3' (SEQ ID NO: 147)
(4) The p35S-Nos plasmid was digested with restriction enzyme HindIII, and the attL1 fragment was inserted. The construct was further digested with EcoRI, and the attL2 fragment was inserted. As a result, vector p35SG was constructed.

### <Construction of Construction vector including a Polynucleotide that Encodes Transcription Repressor Converting Peptide>

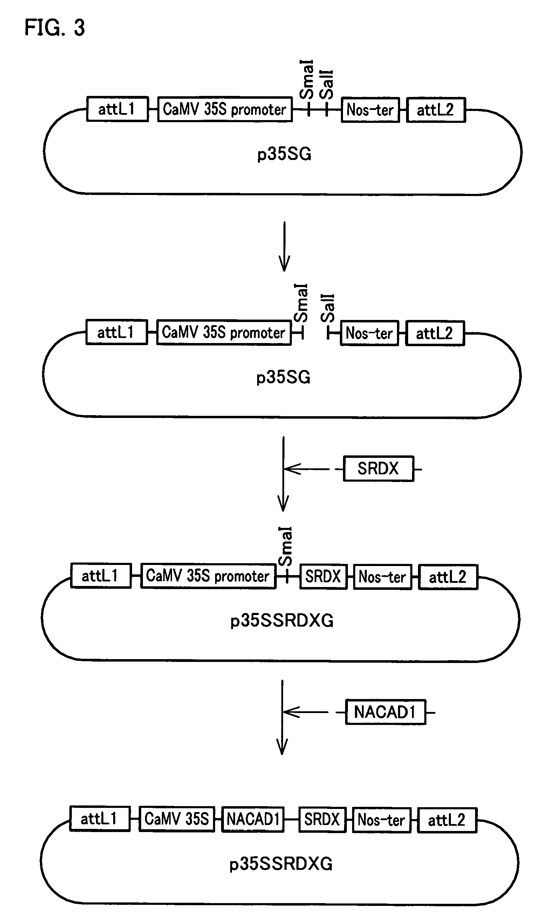

Construction vector p35SSRDXG including a polynucleotide that encodes a transcription repressor converting peptide was constructed according to the following steps (1) and (2) (see Figure 3).
(1) DNA of the sequences below was synthesized. The DNA was designed so that it encodes 12-amino acid peptide LDLDLELRLGFA (SRDX), and that it has an end codon TAA at the 3' end. The DNA was heated at 70°C for 10 minutes, and was allowed to cool and anneal into double strand DNA.
   5'-gggcttgatctggatctagaactccgtttgggtttcgcttaag-3' (SEQ ID NO: 148)
   5'-tcgacttaagcgaaacccaaacggagttctagatccagatcaagccc-3' (SEQ ID NO: 149)
(2) The p35SG was digested with restriction enzymes SmaI and SalI, and the double strand DNA that encodes SRDX was inserted to construct p35SSRDXG.

### <Construction of Transformation vector>

A plant transformation vector pBIGCKH was constructed according to the following steps (1) through (3). The vector pBIGCKH had two att sites to replace the DNA fragment flanked by the att sites of the construction vector.
(1) pBIG provided by Michigan University (Becker, D. Nucleic Acids Res. 18:203, 2990) was digested with restriction enzymes HindIII and EcoI, and GUS and Nos regions were removed by electrophoresis.
(2) Fragment A of the Gateway® vector conversion system purchased from Invitrogen was inserted into the EcoRV site of pBluscript. The construct was digested with HindIII-EcoI, and Fragment A was collected.
(3) The Fragment A was then ligated to the pBIG plasmid fragment, so as to construct pBIGCKH. The vector was able to amplify only in *E. coli* DB3.1 (Invitrogen), and was resistant to chloramphenicol and kanamycin.

### <Incorporation of NACAD1 gene into Construction vector>

A gene that encodes *Arabidopsis thaliana* transcription factor NACAD1 protein was inserted in the construction vector p35SSRDXG according to the following steps (1) through (3).
(1) From the cDNA library prepared with the mRNA obtained from the leaf of *Arabidopsis thaliana,* DNA fragments containing only the coding region of *Arabidopsis thaliana* NACAD1 gene, except for the end codon, were amplified by PCR, using the primers below.
   Primer 1 (NACAD1-F)
   5'-GATGATGTCAAAATCTATGAGCATATC-3' (SEQ ID NO: 155)
   Primer 2 (NACAD1-R)
   5'-TCCACTACCATTCGACACGTGAC-3' (SEQ ID NO: 156)
   The cDNA of NACAD1 gene, and the amino acid sequence encoded thereby are represented by SEQ ID NO: 137 and 136, respectively.
(2) A DNA fragment in the NACAD1 coding region was ligated to the SmaI site of the construction vector p35SSRDXG that had been digested with restriction enzyme SmaI, as shown in Figure 3.
(3) *E. coli* was transformed with the plasmid, and the plasmid was adjusted to determine the base sequence. Then, clones that had inserts in the positive direction were isolated to obtain individuals that had a chimeric gene with SRDX.

The CaMV35S promoter, chimeric gene, Nos-ter, and other DNA fragments on the construction vector were recombined with plant transformation vector pBIGCKH, so as to construct an expression vector that uses a plant as a host. The recombination reaction was performed according to the following steps (1) through (3) with the Gateway® LR clonase® of Invitrogen.
(1) First, to 1.5 µL (about 300 ng) of p35SSRDXG and 4.0 µL (about 600 ng) of pBIGCKH were added 4.0 µL (×5 dilution) of LR buffer and 5.5 µL of TE buffer (10mM TrisCl pH7.0, 1 mM EDTA).
(2) Then, 4.0 µL of LR clonase was added to the solution, and incubation was made at 25°C for 60 minutes. Thereafter, 2 µL of proteinaseK was added, and incubation was made at 37°C for 10 minutes.
(3) *E. coli* (DH5a, etc.) was transformed with 1 to 2 µL of the solution, and selection was made with kanamycin.

### <Production of Plants Transformed with Recombinant Expression Vector>

As detailed in Steps (1) through (3) below, a transgenic plant was produced by transforming *Arabidopsis thaliana* with pBIG-NACAD1SRDX, which is a plasmid that has been prepared by inserting a chimeric gene-containing DNA fragment into pBIGCKH. Transformation of *Arabidopsis thaliana* was carried out according to the procedure described in *Transformation of Arabidopsis thaliana by vacuum infiltration* (http://www.bch.msu.edu/pamgreen/protocol.htm). For the infiltration, no vacuum was used.
(1) The plasmid pBIG-NACAD1SRDX was introduced into *Agrobacterium tumefaciens* strain GV3101 (C58C1Rifr) pM90 (Gmr) (koncz and Schell 1986) by an electroporation method. The cells were cultured to OD600 of 1 in 1 liter of YEP medium containing antibodies (kanamycin (Km) 50 µg/ml, gentamicin (Gm) 25 µg/ml, rifampicillin (Rif) 50 µg/ml). The cells were then removed from the culture medium and suspended in 1 liter of infiltration medium (see Table 2).
(2) In the solution, 14-day-old *Arabidopsis thaliana* was infiltrated for 1 minute to allow for infection. The plants were then grown again to bear seeds. Note that, in the generation infected with Agrobacterium, there is generally no influence of the transforming gene except when it prevents survival of ovule. Thus, dehiscence of anther did not occur and seeds were formed. The seeds were harvested and sterilized for 7 minutes in 25% bleach and 0.02% Triton X-100 solution. After rinsed three times with sterilized water, the seeds were inoculated in sterilized hygromycin selection medium (see Table 3).
(3) From about 2000 seeds inoculated on the hygromycin medium, an average of 50 hygromycin-resistant transformants were obtained. Total RNA was adjusted from these plants, and transfer of NACAD 1 SRDX gene was confirmed by RT-PCR.

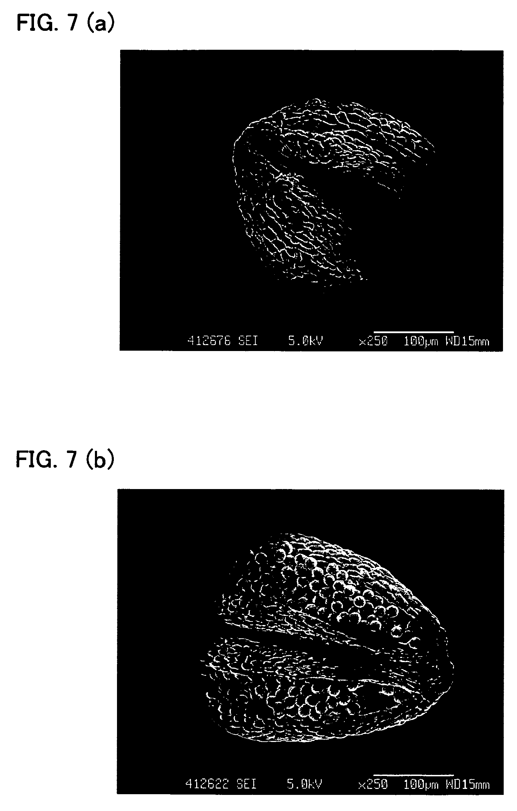

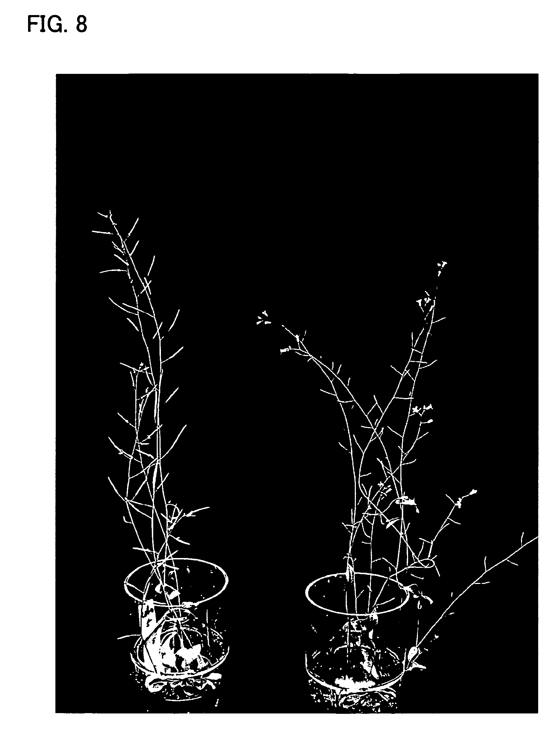

Using a scanning electron microscope (JSM-6330F, JOEL, Ltd.), the shape of anther was observed for several individuals of the transformed plants that were transformed with pBIG-NACAD1SRDX. The result is shown in Figure 7(a). Figure 7(b) shows the shape of anther of wild type of *Arabidopsis thaliana* observed under the scanning electro microscope. As shown in Figure 7(a), dehiscence of anther did not occur in *Arabidopsis thaliana* transformed with pBIG-NACAD1SRDX. It was therefore confirmed that, in *Arabidopsis thaliana* transformed with pBIG-NACAD1SRDX, dehiscence of anther does not occur at all, or occurs only incompletely (though not shown). In fact, the *Arabidopsis thaliana* transformed with pBIG-NACAD1SRDX hardly bore seeds, as can be seen in the right-hand side of Figure 8. The left-hand side of Figure 8 shows wild type *Arabidopsis thaliana.*

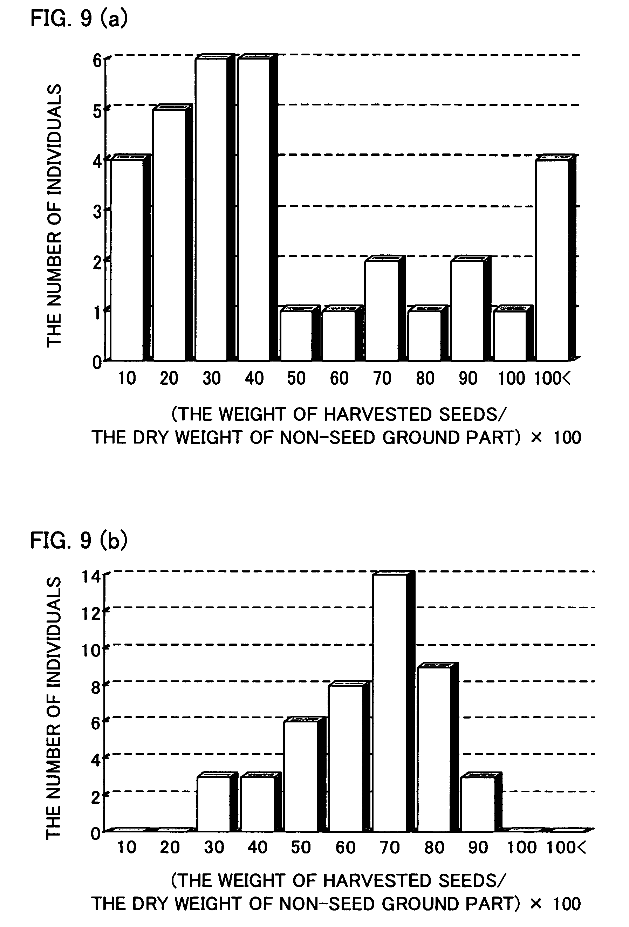

For each of 37 individuals of transformed plants transformed with pBIG-NACAD1SRDX, there was determined a weight proportion of harvested seeds relative to the dry weight of non-seed ground part, and comparisons were made with a group of other types of *Arabidopsis thaliana* that properly formed seeds. Figures 9(a) and 9(b) show the results. In the graphs of Figures 9(a) and 9(b), the vertical axis represents the number of individuals, and the horizontal axis represents class values given by (the weight of harvested seeds/the dry weight of non-seed ground part) × 100. For example, the value 20 on the horizontal axis means that the calculation result of (the weight of harvested seeds/the dry weight of non-seed ground part) × 100 exceeds 10 but does not exceed 20. As shown in Figure 9(a), the plants transformed with pBIG-NACAD1SRDX had more numbers of individuals with a reduced weight proportion of harvested seeds relative to the dry weight of non-seed ground part, as compared with the group of plants that properly formed seeds (Figure 9(b)). As used herein, the "weight of seeds" refers to the total weight of harvested seeds in each individual. The result suggests that the plants transformed with pBIG-NACAD1SRDX can hardly form seeds because dehiscence of anther is suppressed under natural conditions. The seeds obtained in the plants transformed with pBIG-NACAD1SRDX are the result of self-pollination by the pollens that were released from the incompletely dehisced anther.

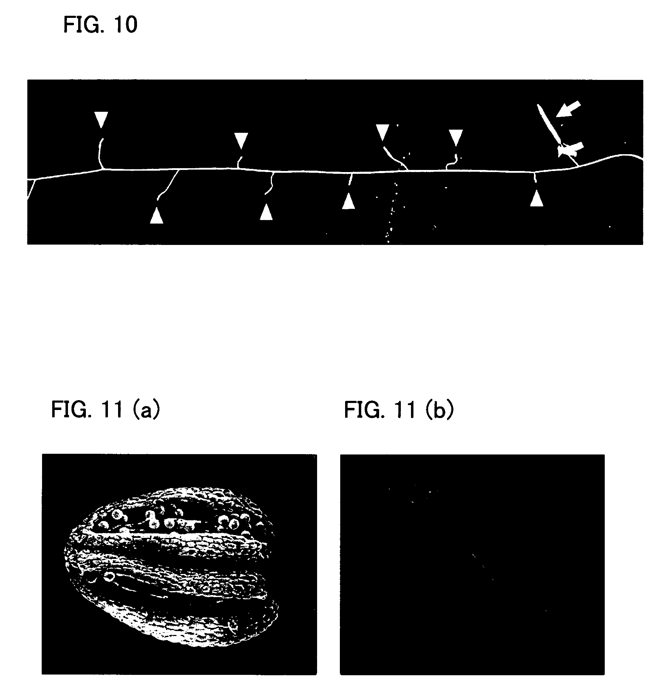

Further, in the plants that were transformed with pBIG-NACAD1SRDX and that have had dehiscence of anther suppressed, pollens were removed from the anther and pollination was attempted to see if it leads to formation of seeds. In Figure 10, the arrow indicates the situation where pollination was made with the pollens that were removed with tweezers from the anther that did not dehisce. Arrow heads indicate positions where the procedure was not performed. As indicated by the arrow in Figure 10, pollination with the pollens led to formation of seeds even when dehiscence of anther was completely suppressed. This shows that the pollen itself was fertile. It was therefore found that the plants transformed with pBIG-NACAD1SRDX cannot form seeds not because the pollens are sterile but because the plants are unable to pollinate due to the suppressed dehiscence of anther. Further, from the fact that pollination of a flower led to formation of seeds even with the dehiscence of anther is suppressed, it was confirmed that the female organ (pistil) was fertile.

### [Example 3]

In this Example, a recombinant expression vector was constructed in which a polynucleotide that encodes 12-amino acid peptide LDLDLELRLGFA (SRDX) (SEQ ID NO: 17), one kind of transcription repressor converting peptide, is ligated to the downstream side of *Arabidopsis thaliana* MYB26 gene between a cauliflower mosaic virus 35S promoter and the transcription end codon of nopaline synthetase gene. The recombinant expression vector was then introduced into *Arabidopsis thaliana* to transform the plant.

### <Construction of Vector for Constructing Transformation vector>

Vector p35SG for constructing a transformation vector was constructed according to the procedure described in Example 2 (see Figure 2).

### <Construction of Construction vector including a Polynucleotide that Encodes Transcription Repressor Converting Peptide>

Construction vector p35SSRDXG including a polynucleotide that encodes a transcription repressor converting peptide was constructed according to the procedure described in Example 2 (see Figure 3).

### <Construction of Transformation vector>

A plant transformation vector pBIGCKH was constructed according to the procedure described in Example 2. The vector pBIGCKH had two att sites to replace the DNA fragment flanked by the att sites of the construction vector.

### <Incorporation of MYB26 gene into Construction vector>

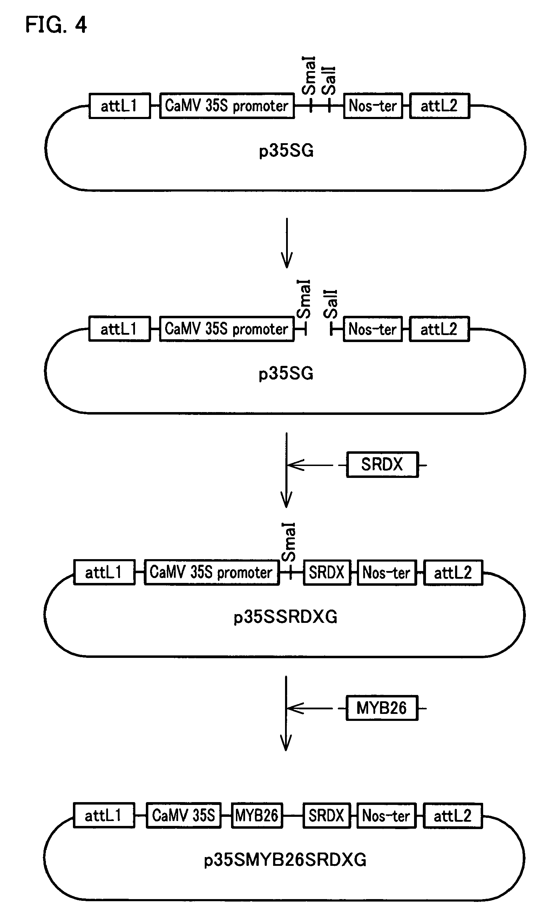

A gene that encodes *Arabidopsis thaliana* transcription factor MYB26 protein was inserted in the construction vector p35SSRDXG according to the following steps (1) through (3).
(1) From the cDNA library prepared with the mRNA obtained from the leaf of *Arabidopsis thaliana,* DNA fragments containing only the coding region of *Arabidopsis thaliana* MYB26 gene, except for the end codon, were amplified by PCR, using the primers below.
   Primer 1 (MYB26-F)
   5'-GATGGGTCATCACTCATGCTGCAACAAGCA-3' (SEQ ID NO: 157)
   Primer 2 (MYB26-R)
   5'-AGTTATGACGTACTGTCCACAAGAGATTGG-3' (SEQ ID NO: 158)
   The cDNA of MYB26 gene, and the amino acid sequence encoded thereby are represented by SEQ ID NO: 139 and 138, respectively.
(2) A DNA fragment in the MYB26 coding region was ligated to the SmaI site of the construction vector p35SSRDXG that had been digested with restriction enzyme SmaI, as shown in Figure 4.
(3) *E. coli* was transformed with the plasmid, and the plasmid was adjusted to determine the base sequence. Then, clones that had inserts in the positive direction were isolated to obtain individuals that had a chimeric gene with SRDX.

### <Construction of Recombinant Expression Vector>

The CaMV35S promoter, chimeric gene, Nos-ter, and other DNA fragments on the construction vector were recombined with plant transformation vector pBIGCKH, so as to construct an expression vector that uses a plant as a host. The recombination reaction was performed with the Gateway® LR clonase® of Invitrogen. The procedure described in <Construction of Recombinant Expression Vector> in Example 2 was followed except that p35SMYB26SRDXG that has incorporated the coding region of MYB26 in the positive direction was used instead of p35SSRDXG.

### <Production of Plants Transformed with Recombinant Expression Vector>

A transgenic plant was produced by transforming *Arabidopsis thaliana* with pBIG-NACAD1SRDX, which is a plasmid that has been prepared by inserting a chimeric gene-containing DNA fragment into pBIGCKH. Transformation of *Arabidopsis thaliana* was carried out according to the procedure described in <Production of Plants Transformed with Recombinant Expression Vector> in Example 2, except that pBIG-MYB26SRDX was used instead of pBIG-NACAD1SRDX.

From about 5000 seeds inoculated on the hygromycin medium, an average of 60 hygromycin-resistant transformants were obtained. Total RNA was adjusted from these plants, and transfer of MYB26SRDX gene was confirmed by RT-PCR.

Using a scanning electron microscope (JSM-6330F, JOEL, LTD.), the shape of anther was observed for several individuals of the transformed plants that were transformed with pBIG-MYB26SRDX. The result is shown in Figure 11(b). Figure 11 (a) shows the shape of anther of wild type *Arabidopsis thaliana* observed under the scanning electro microscope. As shown in Figure 11(b), dehiscence of anther did not occur in *Arabidopsis thaliana* transformed with pBIG-MYB26SRDX. It was therefore confirmed that, in *Arabidopsis thaliana* transformed with pBIG-MYB26SRDX, dehiscence of anther does not occur, or occurs only incompletely (though not shown).

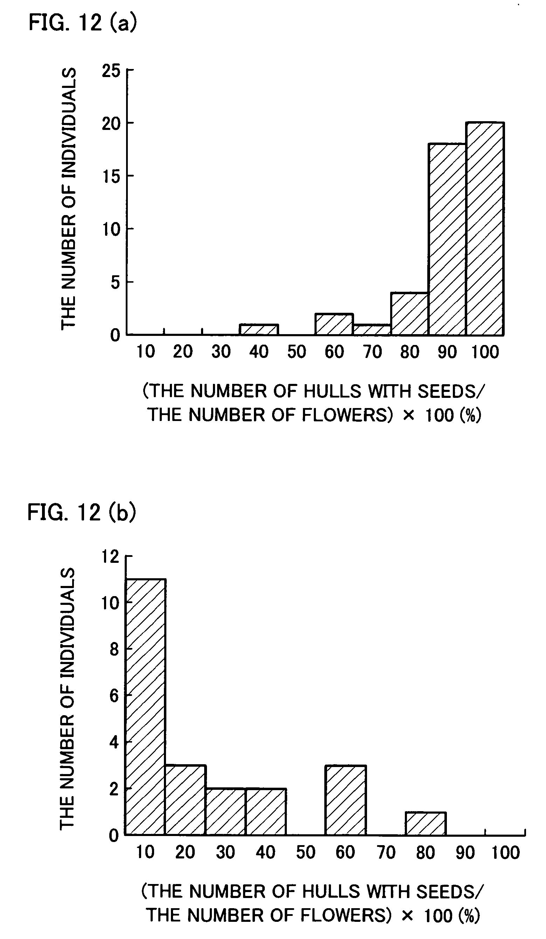

For each of 22 individuals of transformed plants transformed with pBIG-MYB26SRDX, there was determined a proportion of the number of hulls with seeds, relative to the number of flowers, and comparisons were made with a group of other types of *Arabidopsis thaliana* that properly formed seeds. Figure 12 shows the results. In the graphs of Figures 12(a) and 12(b), the vertical axis represents the number of individuals, and the horizontal axis represents class values given by (the number of hulls with seeds/the number of flowers) × 100. For example, the value 20 on the horizontal axis means that the calculation result of (the number of hulls with seeds/the number of flowers) × 100 exceeds 10 but does not exceed 20.

As shown in Figure 12(b), the plants transformed with pBIG-MYB26SRDX had more numbers of individuals with a reduced number of hulls with seeds relative to the number of flowers, as compared with the group of plants that properly formed seeds (Figure 12(a)). The proportion in the number of hulls with seeds relative to the number of flowers was no less than 0 and no more than 10 in 11 out of 22 individuals, indicating that the proportion of individuals that did not form seeds was indeed high. Further, the result suggests that the plants transformed with pBIG-MYB26SRDX can hardly form seeds because dehiscence of anther is suppressed under natural conditions.

The seeds obtained in the plants transformed with pBIG-MYB26SRDX are the result of self-pollination by the pollens that were released from the incompletely dehisced anther. As used herein, the "number of hulls with seeds" refers to the total number of hulls (siliques) with seeds in each individual.

Further, in the plants that were transformed with pBIG-MYB26 and that have had dehiscence of anther suppressed, pollens were removed from the anther and pollination was attempted to see if it leads to formation of seeds. As a result, pollination with the pollens led to formation of seeds even when dehiscence of anther was completely suppressed. This shows that the pollen itself was fertile. It was therefore found that the plants transformed with pBIG-MYB26SRDX cannot form seeds not because the pollens are sterile but because the plants are unable to pollinate due to the suppressed dehiscence of anther. Further, from the fact that pollination of a flower led to formation of seeds even with the dehiscence of anther suppressed, it was confirmed that the female organ (pistil) was fertile.

### [Example 4]

In this Example, a recombinant expression vector was constructed in which a polynucleotide that encodes 12-amino acid peptide LDLDLELRLGFA (SRDX) (SEQ ID NO: 17), one kind of transcription repressor converting peptide, is ligated to the downstream side of *Arabidopsis thaliana* AG gene between a cauliflower mosaic virus 35S promoter and the transcription end codon of nopaline synthetase gene. The recombinant expression vector was then introduced by an Agrobacterium method into *Arabidopsis thaliana* to transform the plant.

### <Construction of Vector for Constructing Transformation vector>

Vector p35SG for constructing a transformation vector was constructed according to the procedure described in Example 2 (see Figure 2).

### <Construction of Construction vector including a Polynucleotide that Encodes Transcription Repressor Converting Peptide>

Construction vector p35SSRDXG including a polynucleotide that encodes a transcription repressor converting peptide was constructed according to the procedure described in Example 2 (see Figure 3).

### <Construction of Transformation vector>

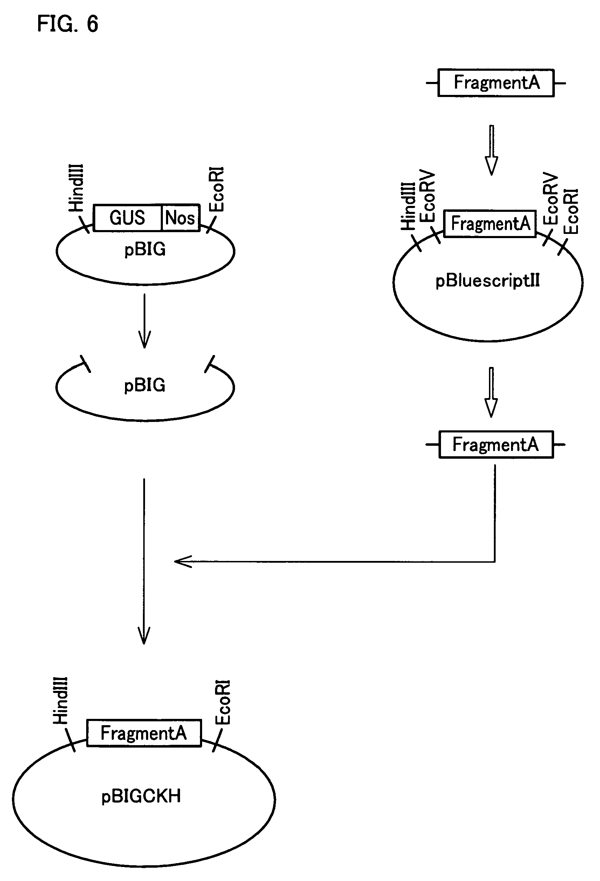

A plant transformation vector pBIGCKH was constructed according to the procedure described in Example 2 (see Figure 6). The vector pBIGCKH had two att sites to replace the DNA fragment flanked by the att sites of the construction vector.

### <Incorporation of AG gene into Construction vector>

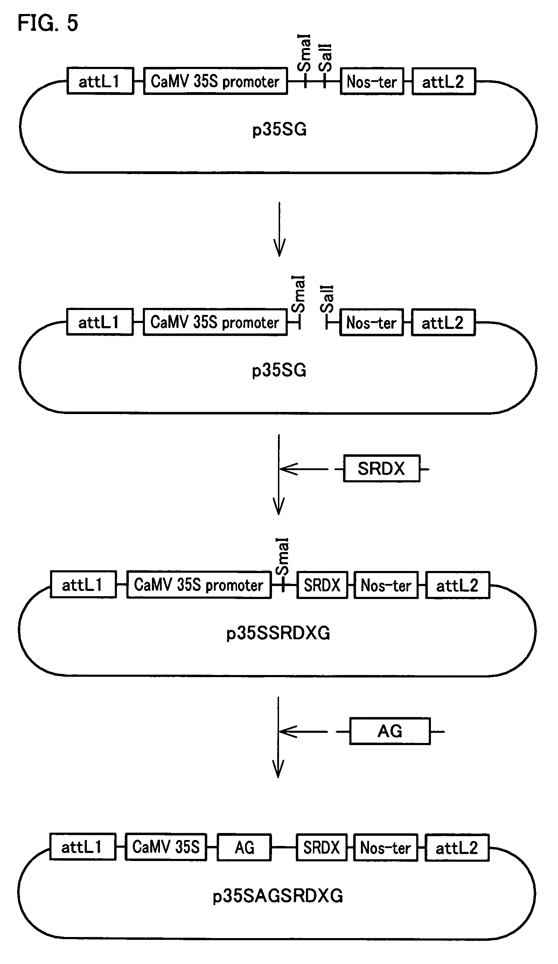

A gene that encodes *Arabidopsis thaliana* transcription factor AG protein was inserted in the construction vector p35SSRDXG according to the following steps (1) through (3).
(1) Using the total length cDNA pda01673 of Arabidopsis thaliana as a template, DNA fragments containing only the coding region of AG polynucleotide (At4g18960), except for the end codon, were amplified by PCR, using the primers below.
   Primer 1 5'-atgaccgcgtaccaatcggagctaggagg-3' (SEQ ID NO: 150)
   Primer 2 5'-cactaactggagagcggtttggtcttggcg-3' (SEQ IDNO: 151)
   The amino acid sequence encoded by AG polynucleotide, and the cDNA of AG polynucleotide are represented by SEQ ID NO: 140 and 141, respectively.
(2) A DNA fragment in the AG coding region was ligated to the SmaI site of the construction vector p35SSRDXG that had been digested with restriction enzyme SmaI, as shown in Figure 5.
(3) *E. coli* was transformed with the plasmid, and the plasmid was adjusted to determine the base sequence. Then, clones that had inserts in the positive direction were isolated to obtain individuals that had a chimeric gene with SRDX.

### <Construction of Recombinant Expression Vector>

The CaMV35S promoter, chimeric gene, Nos-ter, and other DNA fragments on the construction vector were recombined with plant transformation vector pBIGCKH, so as to construct an expression vector that uses a plant as a host. The recombination reaction was performed with the Gateway® LR clonase® of Invitrogen. The procedure described in <Construction of Recombinant Expression Vector> in Example 2 was followed except that p35SAGSRDXG that has incorporated the coding region of AG in the positive direction was used instead of p35SSRDXG.

### <Production of Plants Transformed with Recombinant Expression Vector>

A transgenic plant was produced by transforming *Arabidopsis thaliana* with pBIG-AGSRDX, which is a plasmid that has been prepared by inserting a chimeric gene-containing DNA fragment into pBIGCKH. Transformation of *Arabidopsis thaliana* was carried out according to the procedure described in <Production of Plants Transformed with Recombinant Expression Vector> in Example 2, except that pBIG-AGSRDX was used instead of pBIG-NACAD1SRDX.

From about 5000 seeds inoculated on the hygromycin medium, an average of 50 hygromycin-resistant transformants were obtained. Total RNA was adjusted from these plants, and transfer of AGSRDX gene was confirmed by RT-PCR.

Referring to Figure 13 through Figure 16, description is made below as to the plants transformed with pBIG-AGSRDX. Figure 13(a) shows a flower of *Arabidopsis thaliana* that was transformed with pBIG-AGSRDX and formed double flowers. Figure 13(b) shows a whole part of the double flowered *Arabidopsis thaliana.* As shown in Figure 13(a), the *Arabidopsis thaliana* transformed with pBIG-AGSRDX formed complete double flowers in 16 of 28 individuals.

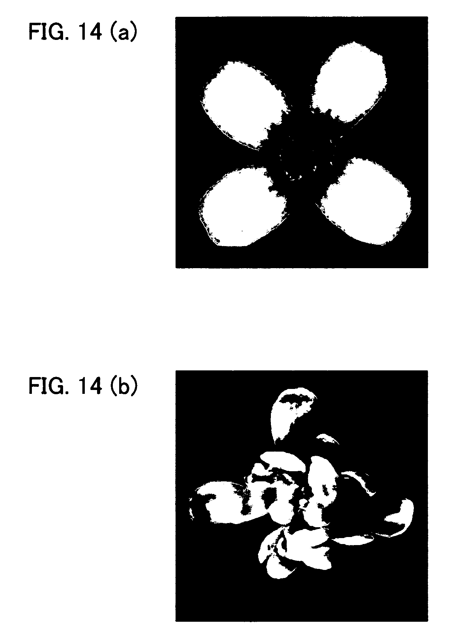

Figure 14(a) shows a flower of wild type *Arabidopsis thaliana.* Figure 14(b) shows a flower of AG mutant *Arabidopsis thaliana.* While the wild type *Arabidopsis thaliana* had 4 sepals, 4 petals, 6 stamens, and 1 pistil, the *Arabidopsis thaliana* transformed according to the method of the present invention had its stamen changed to a petal and had a new flower at whorl 4 where pistil is generally formed. The AG mutants had a similar structure but the *Arabidopsis thaliana* transformed according to the method of the present invention formed more well balanced and beautiful double flowers with a narrower gap between petals, as compared with the AG mutants.

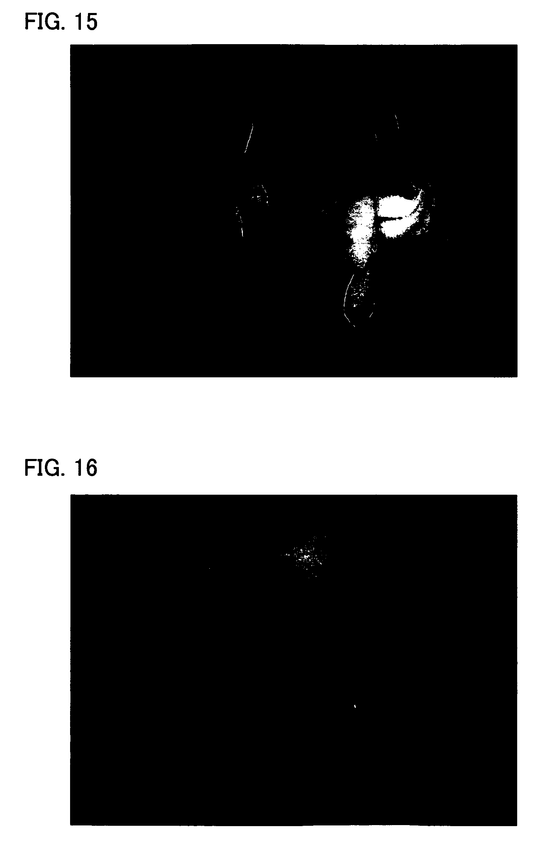

Figure 15 shows a flower of 10 out of 28 individuals of the *Arabidopsis thaliana* transformed with pBIG-AGSRDX. These 10 individuals formed incomplete double flowers. Figure 16 shows a flower in 2 of 28 individuals of the *Arabidopsis thaliana* transformed with pBIG-AGSRDX. These 2 individuals had flower morphology similar to that of the wild type.

The 16 individuals were completely sterile plants with no stamen or pistil. The 10 individuals did not form seeds even though incomplete stamen-like or pistil-like organs were formed. That is, these plants were also sterile. The 2 individuals formed stamen and pistil but produced a very few seeds. In sum, the plants transformed with pBIG-AGSRDX were all sterile.

The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, a producing process of a sterile plant according to the present invention causes a plant to produce a chimeric protein, in which a transcription factor that promotes expression of a gene associated with formation of floral organs is fused with a functional peptide that converts an arbitrary transcription factor into a transcription repressor, so as to suppress expression of the gene associated with formation of floral organs and thereby produce male sterile plants.

Thus, a male sterile plant can be produced by transforming a target plant with a chimeric gene that encodes the chimeric protein. It is therefore possible to easily produce male sterility in a target plant without using complicated genetic recombinant techniques.

Further, a chimeric protein used in the present invention acts dominantly over endogenous genes. Thus, regardless of whether the plant is a haploid or an amphiploid or whether there are functionally redundant genes, a chimeric protein according to the present invention can equally suppress expression of a gene associated with formation of floral organs. It is therefore possible to easily transform any plant into a male sterile plant, provided that the gene is transferable into the plant.

Further, the amino acid sequence of the transcription factor that promotes transcription of a gene associated with formation of floral organs, as used in the present invention, is believed to be highly conserved among plants of many different species. Thus, a chimeric protein constructed in a specific model plant can be introduced into other plants to easily produce male sterile plants in a wide variety of plant species.

Thus, with the present invention, a so-called male sterile plant, which is not capable of normal pollen production yet possesses fertile pistils can be produced in a wide variety of plants. The present invention is therefore applicable and highly useful in agriculture, forestry, agribusiness, processing industries for agricultural products, and food industries.

As described above, a process according to the present invention for producing a plant in which dehiscence of anther is suppressed causes a plant to produce a chimeric protein, in which a transcription factor that promotes expression of a gene associated with formation of floral organs is fused with a functional peptide that converts an arbitrary transcription factor into a transcription repressor, so as to suppress expression of the gene associated with dehiscence of anther and thereby produce a plant in which dehiscence of anther is suppressed.

Thus, a plant in which dehiscence of anther is suppressed can be produced by transforming a target plant with a chimeric gene that encodes the chimeric protein. It is therefore possible to easily suppress dehiscence of anther in a target plant without using complicated genetic recombinant techniques.

Further, a chimeric protein used in the present invention acts dominantly over endogenous genes. Thus, regardless of whether the plant is a haploid or an amphiploid, or whether there are functionally redundant genes, a chimeric protein according to the present invention can equally suppress expression of a gene associated with dehiscence of anther. It is therefore possible to easily transform any plant into a plant in which dehiscence of anther is suppressed, provided that the gene is transferable into the plant.

Further, the amino acid sequence of the transcription factor that promotes transcription of a gene associated with dehiscence of anther, as used in the present invention, is believed to be highly conserved among plants of many different species. Thus, a chimeric protein constructed in a specific model plant can be introduced into other plants to easily produce a plant in which dehiscence of anther is suppressed in a wide variety of plant species.

As described above, a plant in which dehiscence of anther is suppressed can be produced by suppressing expression of a gene associated with dehiscence of anther. The present invention is therefore applicable and highly useful in agriculture, forestry, agribusiness, processing industries for agricultural products, and food industries.

As described above, a process according to the present invention for producing a sterile plant causes a plant to produce a chimeric protein, in which a transcription factor that promotes expression of a gene associated with formation of stamen and pistil is fused with a functional peptide that converts an arbitrary transcription factor into a transcription repressor, so as to suppress transcription of target genes of the transcription factor and thereby produce double flowered plants. It is therefore possible to conveniently and reliably produce double flowered plants in a short time period.

As described above, double flowered plants or sterile plants can be obtained by suppressing transcription of target genes of AG transcription factor. The present invention is therefore applicable and useful in agriculture, horticulture, gardening, and agribusiness, for example.

## Claims

1. A producing process of a sterile plant, comprising causing a plant to produce a chimeric protein, in which a transcription factor that promotes expression of a gene associated with formation of floral organs is fused with a functional peptide that converts an arbitrary transcription factor into a transcription repressor, so as to sterilize the plant.

2. A producing process of a sterile plant, comprising causing a plant to produce a chimeric protein, in which a transcription factor that promotes expression of a gene associated with formation of floral organs is fused with a functional peptide that converts an arbitrary transcription factor into a transcription repressor, so as to suppress expression of the gene associated with formation of floral organs.

3. A producing process of a sterile plant as set forth in claim 1, wherein the transcription factor that promotes expression of a gene associated with formation of floral organs is a transcription factor associated with formation of stamen or pistil.

4. A producing process of a sterile plant as set forth in any one of claims 1 through 3, wherein at least formation of stamen is suppressed in the sterile plant.

5. A producing process of a sterile plant as set forth in claim 3, wherein the transcription factor associated with formation of stamen or pistil is a transcription factor that promotes transcription of a gene associated with dehiscence of anther, and wherein a chimeric protein in which the transcription factor is fused with a functional peptide that converts an arbitrary transcription factor into a transcription repressor is produced in a plant so as to suppress dehiscence of anther.

6. A producing process of a sterile plant as set forth in claim 5, wherein the transcription factor that promotes transcription of a gene associated with dehiscence of anther is a transcription factor with an MYB domain, and wherein a chimeric protein in which the transcription factor is fused with a functional peptide that converts an arbitrary transcription factor into a transcription repressor is produced in a plant so as to suppress transcription of the gene associated with dehiscence of anther.

7. A producing process of a sterile plant as set forth in claim 5 or 6, wherein the plant has sterile female organs.

8. A producing process of a sterile plant as set forth in any one of claims 5 through 7, wherein the plant produces sterile pollens.

9. A producing process of a sterile plant, comprising causing a plant to produce a chimeric protein, in which a transcription factor associated with formation of stamen and pistil is fused with a functional peptide that converts an arbitrary transcription factor into a transcription repressor, so as to produce a double-flowered plant.

10. A producing process of a sterile plant as set forth in any one of claims 1 through 4, comprising a transforming step of introducing into plant cells a recombinant expression vector that includes a chimeric gene containing (i) a coding gene of the transcription factor and (ii) a polynucleotide that encodes the functional peptide.

11. A producing process of a sterile plant as set forth in claim 10, further comprising an expression vector constructing step of constructing the recombinant expression vector.

12. A producing process of a sterile plant as set forth in any one of claims 1, 3, and 5 through 8, comprising a transforming step of introducing into plant cells a recombinant expression vector that includes a chimeric gene containing (i) a coding gene of the transcription factor and (ii) a polynucleotide that encodes the functional peptide.

13. A producing process of a sterile plant as set forth in claim 12, further comprising an expression vector constructing step of constructing the recombinant expression vector.

14. A producing process of a sterile plant as set forth in any one of claims 1, 3, and 9, comprising a transforming step of introducing into plant cells a recombinant expression vector that includes a chimeric gene containing (i) a coding gene of the transcription factor and (ii) a polynucleotide that encodes the functional peptide.

15. A producing process of a sterile plant as set forth in claim 14, further comprising an expression vector constructing step of constructing the recombinant expression vector.

16. A producing process of a sterile plant as set forth in any one of claims 1 through 4, 10, and 11, wherein the transcription factor is:
(e) a protein with an amino acid sequence represented by SEQ ID NO: 134, or
(f) a protein with the substitution, deletion, insertion, and/or addition of one to several amino acids in the amino acid sequence represented by SEQ ID NO: 134, and capable of promoting expression of the gene associated with formation of floral organs.

17. A producing process of a sterile plant as set forth in claim 10 or 11, wherein the coding gene of the transcription factor is:
(e) a gene that has a base sequence of SEQ ID NO: 135 as an open reading frame; or
(f) a gene that hybridizes under stringent conditions with a gene of a base sequence complementary to the gene of the base sequence represented by SEQ ID NO: 135, and that encodes the transcription factor that promotes expression of the gene associated with formation of floral organs.

18. A producing process of a sterile plant as set forth in any one of claims 1, 3, 5, 7, 8, 12, and 13, wherein the transcription factor is:
(a) a protein with an amino acid sequence represented by SEQ ID NO: 136; or
(b) a protein with the substitution, deletion, insertion, and/or addition of one to several amino acids in the amino acid sequence represented by SEQ ID NO: 136, and capable of promoting transcription of a gene associated with dehiscence of anther.

19. A producing process of a sterile plant as set forth in any one of claims 1, 3, 5, 7, 8, 12, and 13, wherein the transcription factor shares 50% or greater homology with the amino acid sequence of SEQ ID NO: 136, and is a protein capable of promoting transcription of a gene associated with dehiscence of anther.

20. A producing process of a sterile plant as set forth in claim 12 or 13, wherein the coding gene of the transcription factor is:
(c) a gene that has a base sequence of SEQ ID NO: 137 as an open reading frame; or
(d) a gene that hybridizes under stringent conditions with a gene of a base sequence complementary to the gene of the base sequence represented by SEQ ID NO: 137, and that encodes a transcription factor that promotes transcription of a gene associated with dehiscence of anther.

21. A producing process of a sterile plant as set forth in any one of claims 1, 3, 6 through 8, 12, and 13, wherein the transcription factor is:
(a) a protein with an amino acid sequence represented by SEQ ID NO: 138; or
(b) a protein with the substitution, deletion, insertion, and/or addition of one to several amino acids in the amino acid sequence represented by SEQ ID NO: 138, and capable to promoting transcription of a gene associated with dehiscence of anther.

22. A producing process of a sterile plant as set forth in claim 12 or 13, wherein the coding gene of the protein is:
(c) a gene that has a base sequence of SEQ ID NO: 139 as an open reading frame; or
(d) a gene that hybridizes under stringent conditions with a gene of a base sequence complementary to the gene of the base sequence represented by SEQ ID NO: 139, and that encodes a transcription factor that promotes transcription of a gene associated with dehiscence of anther.

23. A producing process of a sterile plant as set forth in any one of claims 1, 3, 9, 14 and 15, wherein the transcription factor is:
(a) a protein with an amino acid sequence represented by SEQ ID NO: 140; or
(b) a protein with the substitution, deletion, insertion, and/or addition of one to several amino acids in the amino acid sequence represented by SEQ ID NO: 140.

24. A producing process of a sterile plant as set forth in claim 14 or 15, wherein the coding gene of the transcription factor is:
(c) a gene that has a base sequence of SEQ ID NO: 141 as an open reading frame; or
(d) a gene that hybridizes under stringent conditions with a gene of a base sequence complementary to the gene of the base sequence represented by SEQ ID NO: 141, and that encodes a protein associated with formation of stamen and pistil.

25. A producing process of a sterile plant, said process using a gene that encodes:
(a) a protein with an amino acid sequence represented by SEQ ID NO: 136; or
(b) a protein with the substitution, deletion, insertion, and/or addition of one to several amino acids in the amino acid sequence represented by SEQ ID NO: 136, and capable to promoting transcription of a gene associated with dehiscence of anther, or
said process using:
(c) a gene that has a base sequence of SEQ ID NO: 137 as an open reading frame; or
(d) a gene that hybridizes under stringent conditions with a gene of a base sequence complementary to the gene of the base sequence represented by SEQ ID NO: 137.

26. A producing process of a sterile plant as set forth in any one of claims 1 through 25, wherein the functional peptide has an amino acid sequence represented by one of:
(1) X1-Leu-Asp-Leu-X2-Leu-X3, where X1 represents 0 to 10 amino acid residues, X2 represents Asn or Glu, and X3 represents at least 6 amino acid residues;
(2) Y1-Phe-Asp-Leu-Asn-Y2-Y3, where Y1 represents 0 to 10 amino acid residues, Y2 represents Phe or Ile, and Y3 represents at least 6 amino acid residues;
(3) Z1-Asp-Leu-Z2-Leu-Arg-Leu-Z3, where Z1 represents Leu, Asp-Leu, or Leu-Asp-Leu, Z2 represents Glu, Gln, or Asp, and Z3 represents 0 to 10 amino acid residues; and
(4) Asp-Leu-Z4-Leu-Arg-Leu, where Z4 is Glu, Gln, or Asp.

27. A producing process of a sterile plant as set forth in any one of claims 1 through 25, wherein the functional peptide has an amino acid sequence represented by any one of SEQ ID NO: 1 though 17.

28. A producing process of a sterile plant, wherein the functional peptide is:
(e) a peptide with an amino acid sequence represented by SEQ ID NO: 18 or 19; or
(f) a peptide with the substitution, deletion, insertion, and/or addition of one to several amino acids in the amino acid sequence represented by SEQ ID NO: 18 or 19.

29. A producing process of a sterile plant as set forth in any one of claim 1 through 25, wherein the functional peptide has an amino acid sequence represented by:
α1-Leu-β1-Leu-γ1-Leu (5)
where α1 is Asp, Asn, Glu, Gln, Thr, or Ser, β1 is Asp, Gln, Asn, Arg, Glu, Thr, Ser, or His, and γ1 is Arg, Gln, Asn, Thr, Ser, His, Lys, or Asp.

30. A producing process of a sterile plant as set forth in any one of claim 1 through 25, wherein the functional peptide has an amino acid sequence represented by:
α1-Leu-β1-Leu-γ2-Leu (6)
α1-Leu-β2-Leu-Arg-Leu (7)
α2-Leu-β1-Leu-Arg-Leu (8)
where α1 is Asp, Asn, Glu, Gln, Thr, or Ser, α2 is Asn, Glu, Gln, Thr, or Ser, β1 is Asp, Gln, Asn, Arg, Glu, Thr, Ser, or His, β2 is Asn, Arg, Thr, Ser, or His, and γ2 is Gln, Asn, Thr, Ser, His, Lys, or Asp.

31. A producing process of a sterile plant as set forth in any one of claims 1 through 25, wherein the functional peptide is a peptide with an amino acid sequence represented by SEQ ID NO: 20, 21, 22, 23, 24, 25, 26, 27, 28, 29, 30, 31, 32, 33, 34, 35, 38, 39, 40, or 152.

32. A producing process of a sterile plant as set forth in any one of claims 1 through 25, wherein the functional peptide is a peptide with an amino acid sequence represented by SEQ ID NO: 36 or 37.

33. A sterile plant, which is produced by the producing process of any one of claims 1 through 32.

34. A sterile plant as set forth in claim 33, wherein the sterile plant includes at least one of: an adult plant; a plant cell; a plant tissue; a callus; and a seed.

35. A sterile plant producing kit for performing the producing process of any one of claims 1 through 32, said kit comprising a recombinant expression vector that includes:
a gene that encodes a transcription factor that promotes expression of a gene associated with formation of floral organs, formation of stamen or pistil, dehiscence of anther, or formation of stamen and pistil;
a polynucleotide that encodes a functional peptide that converts an arbitrary transcription factor into a transcription repressor; and
a promoter.

36. A sterile plant producing kit as set forth in claim 35, further comprising chemicals for introducing the recombinant expression vector into plant cells.
